# EUROPEAN PATENT APPLICATION

(11) **EP 4 535 194 A1**
(43) Date of publication of application: **09.04.2025**
(21) Application number: 23815613.7
(22) Date of filing: 19.04.2023
(51) Int. Cl.: G06F 16/9035

(54) **DETERMINATION DEVICE AND DETERMINATION METHOD**

(30) Priority: 01.06.2022 JP 2022089589
(71) Applicant: Sony Group Corporation, Tokyo 108-0075 (JP)
(72) Inventor: SUZUKI, Saya, Tokyo 108-0075 (JP); HIGASHINAKA, Chiaki, Tokyo 108-0075 (JP); KURASAWA, Hiromi, Tokyo 108-0075 (JP)
(74) Representative: 2SPL Patentanwälte PartG mbB
(86) International application number: PCT/JP2023/015557
(87) International publication number: WO 2023/233852

(57) **Abstract**

A determination device according to one aspect according to the present disclosure includes an input unit that receives an input of reaction information indicating a reaction of a user, a generation unit that generates first persona information that is information generated on the basis of the reaction information received by the input unit, the information indicating a characteristic of the user, a determination unit that determines consistency between the first persona information generated by the generation unit and second persona information based on past reaction information of the user, and an update unit that updates the persona information regarding the user on the basis of the consistency determined by the determination unit.

## Description

### Field

The present disclosure relates to a determination device and a determination method for determining consistency of persona information indicating a characteristic of an individual.

### Background

With the development of technologies such as an interaction system based on artificial intelligence (AI), opportunities for interaction between a user and an interaction system in a natural language are increasing. At that time, the interaction system sometimes generates and holds information indicating a characteristic of an individual user in order to generate a natural conversation sentence or executing consistent information processing and the like.

As an example, there is known a technology in which an agent (interaction system) that has a conversation with a user records a content of the conversation and supplementary information regarding the conversation in a database as conversation data (for example, Patent Literature 1). In such technology, the user himself/herself accesses the database, browses the conversation data, and selects favorite information, so that the interaction system performs learning on the basis of the selected favorite information of the user. The interaction system can reflect the learned content in the next conversation.

### Citation List

### Patent Literature

Patent Literature 1: JP 2021-144290 A

### Summary

### Technical Problem

According to the conventional technology, a user can use an agent system capable of performing an interaction to suit his/her taste.

However, in the above-described conventional technology, since the user himself/herself is required to select taste information, in a case where the user's taste and characteristic change, there is a case where the interaction system cannot cope with such change in information unless the user frequently refers to the conversation data. It is supposed that the user's taste and characteristic are updated with a lapse of time, but in the conventional technology, information is not updated in real time unless the user himself/herself performs some selection operation. In this case, in the conventional technology, since old information different from current information is used in an interaction, there is a risk of executing an inappropriate response or processing in which the information does not match.

Therefore, the present disclosure proposes a determination device and a determination method capable of appropriately determining information indicating a characteristic of an individual user.

### Solution to Problem

In order to solve the above problems, the determination device according to one aspect according to the present disclosure includes an input unit that receives an input of reaction information indicating a reaction of a user, a generation unit that generates first persona information that is information generated on the basis of the reaction information received by the input unit, the information indicating a characteristic of the user, a determination unit that determines consistency between the first persona information generated by the generation unit and second persona information based on past reaction information of the user, and an update unit that updates the persona information regarding the user on the basis of the consistency determined by the determination unit.

### Brief Description of Drawings

FIG. 1 is a diagram illustrating an outline of determination processing according to a first embodiment.
FIG. 2 is a diagram illustrating a configuration example of a determination device according to the first embodiment.
FIG. 3 is a diagram illustrating information received by an input unit according to the first embodiment.
FIG. 4 is a diagram illustrating a configuration of a generation unit according to the first embodiment.
FIG. 5 is a flowchart (1) illustrating a flow of the determination processing according to the first embodiment.
FIG. 6 is a flowchart (2) illustrating a flow of the determination processing according to the first embodiment.
FIG. 7 is an example of a conceptual diagram of a model configuration regarding generation processing of persona information.
FIG. 8 is an example of a conceptual diagram of a model configuration regarding determination processing of persona information.
FIG. 9 is a diagram (1) illustrating a specific example of the determination processing according to the first embodiment.
FIG. 10 is a diagram (2) illustrating a specific example of the determination processing according to the first embodiment.
FIG. 11 is a diagram (3) illustrating a specific example of the determination processing according to the first embodiment.
FIG. 12 is a diagram (4) illustrating a specific example of the determination processing according to the first embodiment.
FIG. 13 is a diagram (5) illustrating a specific example of the determination processing according to the first embodiment.
FIG. 14 is a diagram (6) illustrating a specific example of the determination processing according to the first embodiment.
FIG. 15 is a diagram (7) illustrating a specific example of the determination processing according to the first embodiment.
FIG. 16 is a diagram (8) illustrating a specific example of the determination processing according to the first embodiment.
FIG. 17 is a diagram (9) illustrating a specific example of the determination processing according to the first embodiment.
FIG. 18 is a diagram (10) illustrating a specific example of the determination processing according to the first embodiment.
FIG. 19 is a diagram (11) illustrating a specific example of the determination processing according to the first embodiment.
FIG. 20 is a diagram (12) illustrating a specific example of the determination processing according to the first embodiment.
FIG. 21 is a diagram (13) illustrating a specific example of the determination processing according to the first embodiment.
FIG. 22 is a diagram (14) illustrating a specific example of the determination processing according to the first embodiment.
FIG. 23 is a diagram (15) illustrating a specific example of the determination processing according to the first embodiment.
FIG. 24 is a diagram (16) illustrating a specific example of the determination processing according to the first embodiment.
FIG. 25 is a diagram (17) illustrating a specific example of the determination processing according to the first embodiment.
FIG. 26 is a diagram (18) illustrating a specific example of the determination processing according to the first embodiment.
FIG. 27 is a diagram (19) illustrating a specific example of the determination processing according to the first embodiment.
FIG. 28 is a diagram (20) illustrating a specific example of the determination processing according to the first embodiment.
FIG. 29 is a diagram (21) illustrating a specific example of the determination processing according to the first embodiment.
FIG. 30 is a diagram (22) illustrating a specific example of the determination processing according to the first embodiment.
FIG. 31 is a diagram (23) illustrating a specific example of the determination processing according to the first embodiment.
FIG. 32 is a diagram (24) illustrating a specific example of the determination processing according to the first embodiment.
FIG. 33 is a diagram illustrating a configuration example of a determination device according to a second embodiment.
FIG. 34 is a diagram (1) illustrating a specific example of determination processing according to the second embodiment.
FIG. 35 is a diagram (2) illustrating a specific example of the determination processing according to the second embodiment.
FIG. 36 is a diagram illustrating a configuration example of a determination device according to a third embodiment.
FIG. 37 is a diagram (1) illustrating a specific example of determination processing according to the third embodiment.
FIG. 38 is a diagram (2) illustrating a specific example of determination processing according to the third embodiment.
FIG. 39 is a diagram (3) illustrating a specific example of determination processing according to the third embodiment.
FIG. 40 is a diagram (4) illustrating a specific example of determination processing according to the third embodiment.
FIG. 41 is a diagram (5) illustrating a specific example of determination processing according to the third embodiment.
FIG. 42 is a diagram (6) illustrating a specific example of determination processing according to the third embodiment.
FIG. 43 is a diagram (7) illustrating a specific example of determination processing according to the third embodiment.
FIG. 44 is a diagram (8) illustrating a specific example of determination processing according to the third embodiment.
FIG. 45 is a diagram illustrating a configuration example of a determination device according to a fourth embodiment.
FIG. 46 is a diagram illustrating a specific example of determination processing according to the fourth embodiment.
FIG. 47 is a diagram illustrating a configuration example of a determination device according to a fifth embodiment.
FIG. 48 is a diagram (1) illustrating a specific example of determination processing according to the fifth embodiment.
FIG. 49 is a diagram (2) illustrating a specific example of determination processing according to the fifth embodiment.
FIG. 50 is a diagram (3) illustrating a specific example of determination processing according to the fifth embodiment.
FIG. 51 is a hardware configuration diagram illustrating an example of a computer that implements a function of the determination device.

### Description of Embodiments

Hereinafter, embodiments are described in detail with reference to the drawings. Note that, in each of the following embodiments, the same parts are denoted by the same reference signs, and redundant description is omitted.

The present disclosure is described according to the following order of items.
1. First Embodiment
   1-1. Outline of Determination Processing according to First Embodiment
   1-2. Configuration of Determination Device according to First Embodiment
   1-3. Procedure of Determination Processing according to First Embodiment
   1-4. Specific Example of Determination Processing according to First Embodiment
2. Second Embodiment
3. Third Embodiment
4. Fourth Embodiment
5. Fifth Embodiment
6. Variation
   6-1. Device Configuration
   6-2. Reaction Information
7. Other Embodiment
8. Effect of Determination Device according to Present Disclosure
9. Hardware Configuration

### (1. First Embodiment)

### (1-1. Outline of Determination Processing according to First Embodiment)

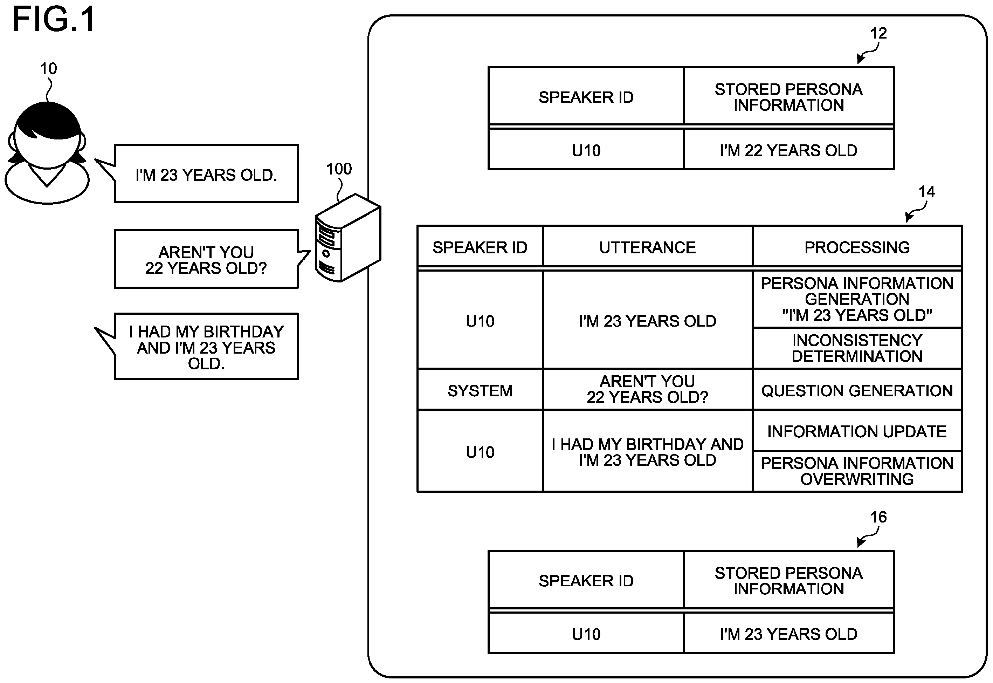

FIG. 1 is a diagram illustrating an outline of determination processing according to a first embodiment. The determination processing according to the first embodiment is implemented by a determination device 100 illustrated in FIG. 1.

The determination device 100 is an example of an information processing device that executes the determination processing according to the embodiment. For example, the determination device 100 is a cloud server, a personal computer (PC), a smartphone, a tablet terminal and the like connected to a network. Note that, the determination device 100 may be a smart home appliance such as a television, a video game console such as a game machine and the like as long as this is an information device having a function to be described later. In an example in FIG. 1, the determination device 100 is an information processing device including a voice agent capable of interacting with a user 10.

The user 10 is a user who uses a voice agent system (hereinafter, sometimes referred to as an "interaction system") provided by the determination device 100. The interaction system provided by the determination device 100 performs, for example, an interaction with the user 10 (text chat, voice chat and the like) or controls execution of various applications in the determination device 100 on the basis of a voice command issued from the user 10.

At that time, the determination device 100 generates and holds information indicating a characteristic of the user 10 in order to generate a natural conversation sentence in the interaction with the user 10 or execute consistent information processing and the like. For example, the determination device 100 holds attribute information such as age and sex of the user 10, interest information such as hobby and taste of the user 10, behavior information such as a future schedule and a past behavior history and the like, and uses the information for interaction. The information indicating a characteristic of an individual is referred to as persona information, and is used in the interaction system and an information transmission system (for example, an advertisement distribution system and the like) via a network.

Note that, in the present disclosure, the persona information widely includes various types of information such as personal information of a target, a relationship between the target and others or objects such as belonging, personal relationship, and possessions of the target, personality of the target appearing in hobby, taste, thought, ideal, impression, feeling and the like, or experience of the target. The persona information may include information optionally defined by an administrator of the determination device 100, the user 10 and the like. A format of the generated persona information may be a natural sentence, a natural sentence with a category tag, a triplet including (speaker, relationship, and item) and the like, but the format is not especially limited. In any format, the persona information is associated with speaker information (identification information (name or ID) for specifying the user and the like) having the persona. Note that, the present disclosure illustrates an example in which the persona information is generated as a natural sentence. When generating the persona information on the basis of the interaction and the like, various known methods (as an example, a method disclosed in Non-Patent Literature "Beyond Goldfish Memory: Long-Term Open-Domain Conversation" (https://parl.ai/projects/sea/) and the like) may be used.

By holding the persona information, the interaction system can perform an interaction according to the taste of the user who is an interaction partner and a natural interaction according to the attribute and schedule of the user.

In contrast, it can be said that the persona information of the user constantly changes depending on a lapse of time, a change in user's state of mind and the like. However, once the interaction system holds the persona information, it is difficult for the interaction system to automatically change the persona information unless there is an explicit change from the user. Therefore, in the interaction system, there is a possibility that a plurality of different pieces of information intervenes as an item that should be unique (such as the age of the user), or inconsistent pieces of information (for example, persona information such as "I like sports" and "I don't like sports") are simultaneously held for the same item. When a smooth conversation between the user and the interaction system is hindered as a result, there is a possibility that a frequency at which the user uses the interaction system decreases or a satisfaction degree of the user with the interaction system decreases. That is, when using the persona information, the interaction system desirably determines whether the persona information of the user is consistent, and appropriately updates the persona information.

Therefore, the determination device 100 according to the present disclosure solves the above-described problem by the following determination processing. That is, in a case of generating the persona information that is the information indicating the characteristic of the user (referred to as "first persona information" for distinction), the determination device 100 refers to the persona information held in the past (referred to as "second persona information" for distinction). Then, the determination device 100 determines consistency between the first persona information and the second persona information, and updates the persona information related to the user on the basis of the determined consistency. As a result, the determination device 100 can appropriately update the persona information of the user without requiring a labor for the user to manually update the information and the like.

Hereinafter, the outline of the determination processing according to the embodiment is described following a flow illustrated in FIG. 1. FIG. 1 illustrates a situation in which the determination device 100 interacts with the user 10 using the interaction system provided in the device itself. Note that, prior to this interaction, the determination device 100 holds persona information "I'm 22 years old" of the user 10 in a data table 12 in which the persona information of the user 10 is stored.

In the example in FIG. 1, the determination device 100 receives an input of a reaction input from the user 10 (uttered voice of the user 10 in this example) in the interaction with the user 10. The determination device 100 converts the received voice into a format that can be input to a learned natural language model (hereinafter, referred to as a "model") used for generating the persona information. For example, the determination device 100 converts the voice into text data, and inputs the text data to a model having text data as an input and persona information as an output.

For example, when the user 10 utters a voice of "I'm 23 years old", the determination device 100 determines that such voice is a sentence, and then generates the persona information corresponding to such sentence. Specifically, the determination device 100 generates the persona information "I'm 23 years old" of the user 10 from the text corresponding to such voice. The determination device 100 holds the generated persona information in a processing table 14.

At that time, the determination device 100 determines that there is past persona information indicating the age of the user 10 and that the past persona information and the persona information generated at the present time are inconsistent with each other with reference to the data table 12. Specifically, when the attribute information (age) of the user 10 that should be unique is different between two pieces of persona information, the determination device 100 determines that there is "inconsistency".

When determining that there is inconsistency, the determination device 100 generates a question for confirming inconsistent information. For example, the determination device 100 generates a sentence with which an answer regarding inconsistent information is estimated to be obtained from the user 10. Specifically, the determination device 100 generates a sentence "Aren't you 22 years old?" with the inconsistent information as an object, which is a question sentence configured to ask the user 10 about such information. The determination device 100 outputs the generated sentence as a voice.

In response to this, the user 10 utters a voice of "I had my birthday and I'm 23 years old". The determination device 100 inputs a text corresponding to the voice into the model, and confirms that the user 10 had a birthday and there is no inconsistency in an increase in age accordingly.

When such information is completed, the determination device 100 updates the persona information in the processing table 14. Specifically, the determination device 100 overwrites the persona information "I'm 22 years old" regarding the attribute of the user 10 with "I'm 23 years old". Then, the determination device 100 stores the overwritten information in a data table 16.

As described above, in a case where the user 10 inputs the reaction information, the determination device 100 generates the first persona information for the reaction information and determines the consistency with the held second persona information. In a case where there is inconsistency, the determination device 100 automatically generates a question for resolving such inconsistency. Then, the determination device 100 updates the persona information or discards the first persona information on the basis of information obtained from the answer to the question. That is, the determination device 100 updates the persona information of the user 10 in real time while interacting with the user 10. As a result, the determination device 100 can continue the interaction using the constantly updated persona information in the interaction with the user 10, so that this can appropriately respond to the user 10.

Note that, although the data table 12, the data table 16, the processing table 14 and the like are illustrated in FIG. 1, the data formats are illustrated for description, and the determination device 100 may hold the persona information in any format.

Although FIG. 1 illustrates the example in which the user 10 utters the voice, an input means is not limited thereto. For example, the input (reaction information of the user 10) to the determination device 100 is not limited to the voice, but may be a text, a gesture, a behavior (for example, selection on a user interface provided by the determination device 100 and the like), a line of sight, a brain wave signal and the like.

### (1-2. Configuration of Determination Device according to First Embodiment)

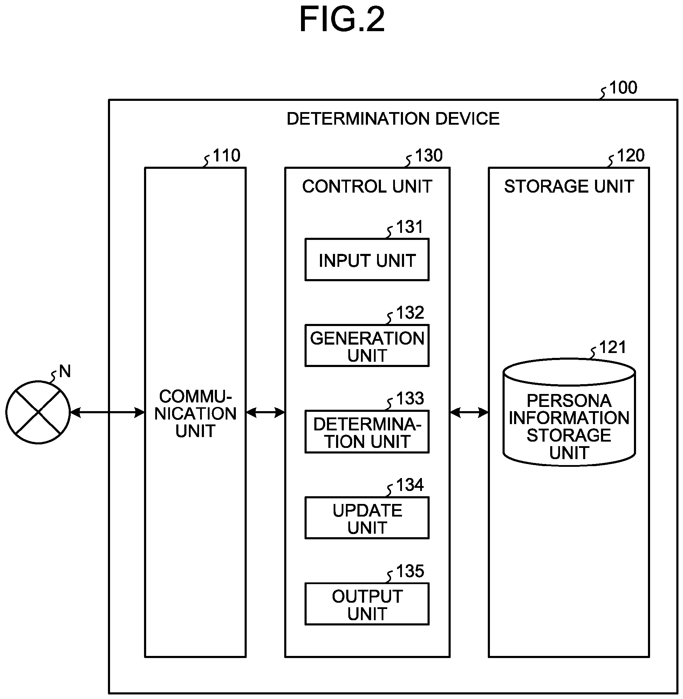

Next, a configuration of the determination device 100 is described. FIG. 2 is a diagram illustrating a configuration example of the determination device 100 according to the embodiment.

As illustrated in FIG. 2, the determination device 100 includes a communication unit 110, a storage unit 120, and a control unit 130. Note that, the determination device 100 may include an input unit (for example, a keyboard, a touch display and the like) that receives various operations from the administrator and the like who manages the determination device 100, and a display unit (for example, a liquid crystal display and the like) for displaying various types of information.

The communication unit 110 is implemented by, for example, a network interface card (NIC), a network interface controller and the like. The communication unit 110 is connected to a network N by wire or wirelessly, and transmits and receives information to and from an external device and the like via the network N. The network N is implemented by, for example, a wireless communication standard or system such as Bluetooth (registered trademark), the Internet, Wi-Fi (registered trademark), ultra wide band (UWB), and low power wide area (LPWA).

The storage unit 120 is implemented by, for example, a semiconductor memory element such as a random access memory (RAM) and a flash memory, or a storage device such as a hard disk and an optical disk.

The storage unit 120 stores various types of information for performing the determination processing according to the embodiment. The storage unit 120 may store the natural language model for generating the persona information from an input text data, the natural language model for generating a question and the like.

The storage unit 120 includes a persona information storage unit 121. The persona information storage unit 121 stores various types of persona information associated with the user (speaker).

The control unit 130 is implemented by, for example, a central processing unit (CPU), a micro processing unit (MPU), GPU and the like executing a program (for example, a determination program according to the present disclosure) stored in the determination device 100 using a random access memory (RAM) and the like as a work area. The control unit 130 is a controller, and may be implemented by, for example, an integrated circuit such as an application specific integrated circuit (ASIC) and a field programmable gate array (FPGA).

As illustrated in FIG. 2, the control unit 130 includes an input unit 131, a generation unit 132, a determination unit 133, an update unit 134, and an output unit 135.

The input unit 131 receives the input of the reaction information indicating the reaction of the user. For example, the input unit 131 receives the input of the voice uttered by the user or the text corresponding to the voice as the reaction information.

The input unit 131 may receive an input of image information obtained by imaging the user together with the reaction information. For example, the input unit 131 controls a camera provided in a display and the like facing the user, acquires an image obtained by imaging the user, and receives the acquired image.

The input unit 131 may receive an input of biological information detected from the user together with the reaction information. For example, the input unit 131 controls various sensors provided in a wearable device worn by the user, and receives biological information such as a perspiration amount and a pulse of the user.

Note that, the input unit 131 may receive not only the voice but also a text input by the user by any input means.

Moreover, the input unit 131 may receive not only the voice but also behavior information on the network of the user as the reaction information. The behavior information on the network of the user is, for example, a browsing behavior or a purchasing behavior on the web network of the user, behavior information in an application such as a game used by the user or the like. These behaviors can be handled similarly to the voice uttered by the user and the like. For example, in a case where the user purchases a product on the web network, the determination device 100 can handle such behavior information similarly to a fact that a text or voice of "A product is purchased on the web network" is input. As a result, the determination device 100 can generate the persona information indicating that the user purchases a product, the user likes the product and the like, without explicitly inputting the voice or text by the user.

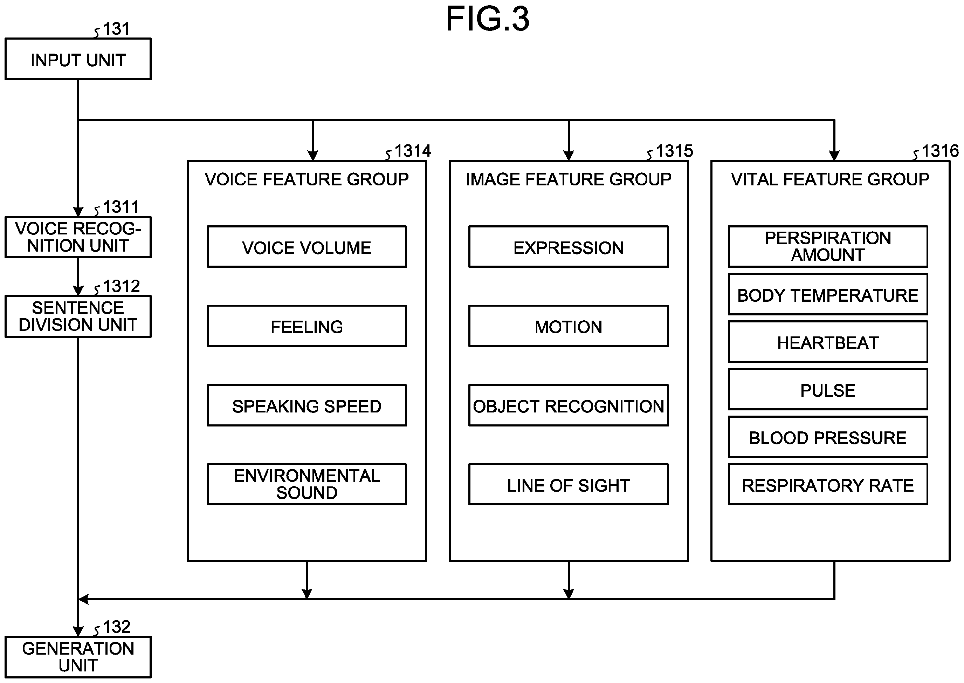

The information received by the input unit 131 will be illustrated with reference to FIG. 3. FIG. 3 is a diagram illustrating the information received by the input unit 131 according to the first embodiment.

As illustrated in FIG. 3, the input unit 131 includes a voice recognition unit 1311 and a sentence division unit 1312.

When receiving a voice, the voice recognition unit 1311 gives speaker information (user ID and the like) to the voice using a known speaker recognition technology. Then, the voice recognition unit 1311 converts the recognized voice into text data. Note that, the input text may be an interaction sequence by a plurality of speakers.

The sentence division unit 1312 divides the input text into one sentence, which is a processing unit of the model. In a case where the input is the interaction sequence, the sentence division unit 1312 may divide the input text into speaker units.

The input unit 131 receives an input of various types of information illustrated in FIG. 3 together with the voice and text. For example, the input unit 131 receives a volume of the voice uttered by the user, a feeling of the user estimated from the voice, a conversation speed in the voice, a surrounding environmental sound and the like included in a voice feature group 1314.

For example, the input unit 131 measures the volume of the utterance using a sensor such as a microphone, estimates feeling information that can be estimated by inputting the voice to a predetermined model, and outputs a score for each feeling. Alternatively, the input unit 131 measures the speed at which the user speaks or estimates a type of the environmental sound included in the voice. The generation unit 132 in a subsequent stage might generate the persona information of the user by using these pieces of information.

The input unit 131 may receive expression, motion, and a line of sight of the user estimated from the image, object information included in the image information and the like included in an image feature group 1315. For example, the input unit 131 measures a score of the expression (for example, a score of joy expression, a score of sorrow expression and the like) from the image obtained by imaging the user by a predetermined expression recognition technology. Alternatively, the input unit 131 estimates the motion of the user appearing on a screen by motion recognition of the camera. The input unit 131 receives object information appearing in the image by using an object recognition technology in the image. The input unit 131 measures the line of sight of the user using a line-of-sight detection technology, detects where the user of which score is counted watches, and receives detected information.

The input unit 131 receives inputs of the perspiration amount, body temperature, heart rate, pulse rate, blood pressure, respiratory rate and the like of the user who is interacting included in a vital feature group 1316 using various sensors.

Note that, the information illustrated in FIG. 3 is an example, and the determination device 100 may receive various types of information that can be sensed in addition to the information illustrated in FIG. 3.

Returning to FIG. 2, the description is continued. The generation unit 132 generates the first persona information that is the information generated on the basis of the reaction information received by the input unit 131 and indicates the characteristic of the user.

Note that, the generation unit 132 may generate the persona information on the basis of various types of information received by the input unit 131 together with the reaction information. For example, the generation unit 132 may generate the first persona information on the basis of at least one of the volume of the voice uttered by the user, the feeling of the user estimated from the voice, the conversation speed in the voice, or the surrounding environmental sound.

Alternatively, the generation unit 132 may generate the first persona information using at least one of the expression, motion, and line of sight of the user estimated from the image information, or the object information included in the image information.

Alternatively, the generation unit 132 may generate the first persona information by using at least one piece of information of the perspiration amount, body temperature, heartbeat, pulse, blood pressure, or respiratory rate of the user measured as the biological information of the user.

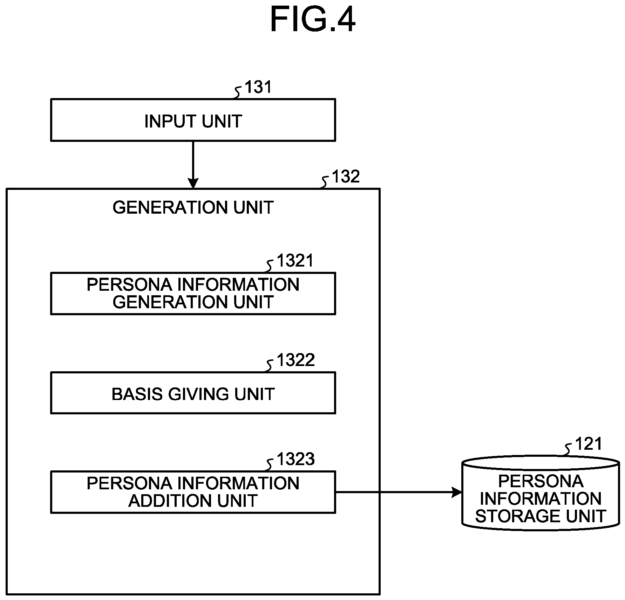

Herein, a configuration of the generation unit 132 is described with reference to FIG. **4.** FIG. 4 is a diagram illustrating the configuration of the generation unit 132 according to the first embodiment. As illustrated in FIG. 4, the generation unit 132 includes a persona information generation unit 1321, a basis giving unit 1322, and a persona information addition unit 1323.

The persona information generation unit 1321 detects the persona information of the speaker (user) on the basis of the information received by the input unit 131, and expresses the information with a simple sentence. In a case where a plurality of pieces of information is included in the input, the persona information generation unit 1321 generates a plurality of pieces of persona information. The persona information may be automatically generated by a machine learning model as described above, or may be automatically generated by rule processing.

The basis giving unit 1322 gives, to the generated persona information, information serving as a basis for generating the persona information. For example, the basis giving unit 1322 gives text data as basis information. Alternatively, the basis giving unit 1322 may give, as the basis information, a sensing value, a vector, a statistical value, an image and the like detected by the sensor when the persona information is generated. The basis giving unit 1322 may give a plurality of bases to one piece of persona information.

The persona information addition unit 1323 adds the persona information generated by the persona information generation unit 1321 to the persona information storage unit 121. Note that, the persona information addition unit 1323 may display the generated persona information on a display or display information obtained by classifying the persona information in order to clearly indicate the same to the administrator and the like of the determination device 100. The classification of the persona information is classification of the information indicated by the persona information for each topic, and is, for example, the attribute information of the user, behavior history of the user, hobby/taste information of the user, a type of content indicated by the persona information and the like. Topic classification may be performed by rule processing, or automatic classification by a machine learning model that has learned the topic classification may be performed. Note that, the topic may be automatically defined by a method such as clustering, or may be optionally designated by the administrator and the like of the determination device 100.

Returning to FIG. 2, the description is continued. The determination unit 133 determines the consistency between the first persona information generated by the generation unit 132 and the second persona information based on the past reaction information of the user. That is, in order to maintain the consistency of the persona information corresponding to the user, the determination unit 133 determines whether inconsistency and the like occurs between the second persona information stored in the persona information storage unit 121 and the first persona information newly generated by the generation unit 132.

For example, the determination unit 133 extracts the second persona information of the same classification as that of the first persona information, and compares contents of the respective pieces of information. Such comparison may be performed, for example, by performing semantic determination of each sentence using the language processing model, or may be performed by sequentially determining all pieces of persona information classified into the same attribute.

As the determination of the consistency, the determination unit 133 determines, for example, whether the pieces of information to be compared are inconsistent with each other, whether they are independent of each other, whether they coincide, or whether the information can be updated to newly generated information.

Inconsistency indicates that there is inconsistency between the second persona information stored in the persona information storage unit 121 and the first persona information newly generated by the generation unit 132.

Information independency indicates that the second persona information stored in the persona information storage unit 121 and the first persona information newly generated by the generation unit 132 are independent of each other.

Information coincidence indicates that the second persona information stored in the persona information storage unit 121 coincides with the first persona information newly generated by the generation unit 132, and the second persona information stored otherwise and the first persona information are in an independent relationship.

Information updatable indicates that, although there is inconsistency between any piece of the second persona information stored in the persona information storage unit 121 and the first persona information newly generated by the generation unit 132, it is possible to directly determine information addition, information overwriting, or information deletion from the interaction with the user (or the interaction between the user and another user). Note that, in this case, any two or more of the information addition, information overwriting, and information deletion might occur simultaneously.

Consistency determination of the persona information described above may be performed by the machine learning model related to language processing for performing semantic determination of the sentence, or may be rule processing. As an example of a mechanism for determining inconsistency, independent information, and information coincidence of the persona information, there is inconsistency detection using natural language inference. The natural language inference is a method of inferring any one of the inconsistency, entailment, and no relationship regarding a relationship between two texts. For example, in "Generating Persona Consistent Dialogues by Exploiting Natural Language Inference (AAAI-20)", natural language inference regarding persona information is exemplified. Note that, the determination device 100 can perform consistency determination using various known technologies in addition to the above-described method.

In a case where there is the inconsistency between the first persona information and the second persona information, the determination unit 133 may transmit a question regarding the inconsistency to the user, and determine the consistency on the basis of an answer of the user.

For example, the determination unit 133 generates a question asking whether target information is correct regarding the target information determined to be inconsistent, and outputs the generated question. Note that, in a case where the user and another user interact with each other, the determination unit 133 may generate a question on the basis of an indication of the inconsistency from the other user.

The update unit 134 updates the persona information related to the user on the basis of the consistency determined by the determination unit 133.

For example, the update unit 134 determines whether to store the first persona information generated by the generation unit 132 in the persona information storage unit 121 on the basis of the consistency determined by the determination unit 133 and the answer of the user to the question.

For example, the update unit 134 determines any one of the information addition, information addition rejection, information update, and information deletion for the first persona information.

The information addition indicates that the first persona information is newly added to the persona information storage unit 121. The information addition rejection indicates that the first persona information is not added to the persona information storage unit 121 and current state is maintained. The information overwriting indicates that the persona information stored in the persona information storage unit 121 is overwritten. The information deletion indicates that the persona information stored in the persona information storage unit 121 is deleted.

For example, in a case where the compared pieces of information are independent of each other, the update unit 134 determines the information addition, and stores both pieces of information in the persona information storage unit 121. Alternatively, in a case where the determination unit 133 determines that the first persona information and the second persona information are not consistent, the update unit 134 holds either the first persona information or the second persona information as the persona information of the user, and discards the persona information that is not held.

Note that, the administrator of the determination device 100 may select to set in advance the processing executed by the update unit 134 so as to perform any of the information addition, information update, and information addition rejection.

The output unit 135 outputs the persona information held in the persona information storage unit 121 in response to various pieces of processing and requests. Note that, when outputting, the output unit 135 may convert into a format different from a persona information format at the time of generation to output.

### (1-3. Procedure of Determination Processing according to First Embodiment)

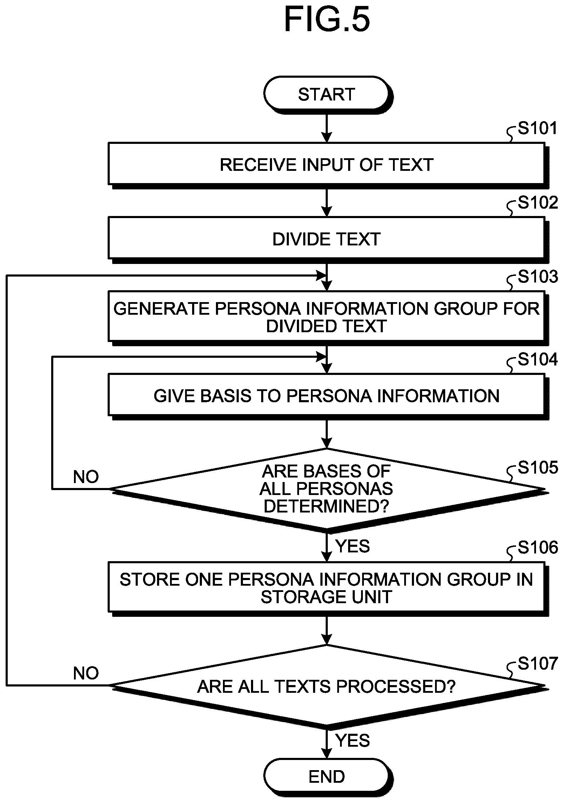

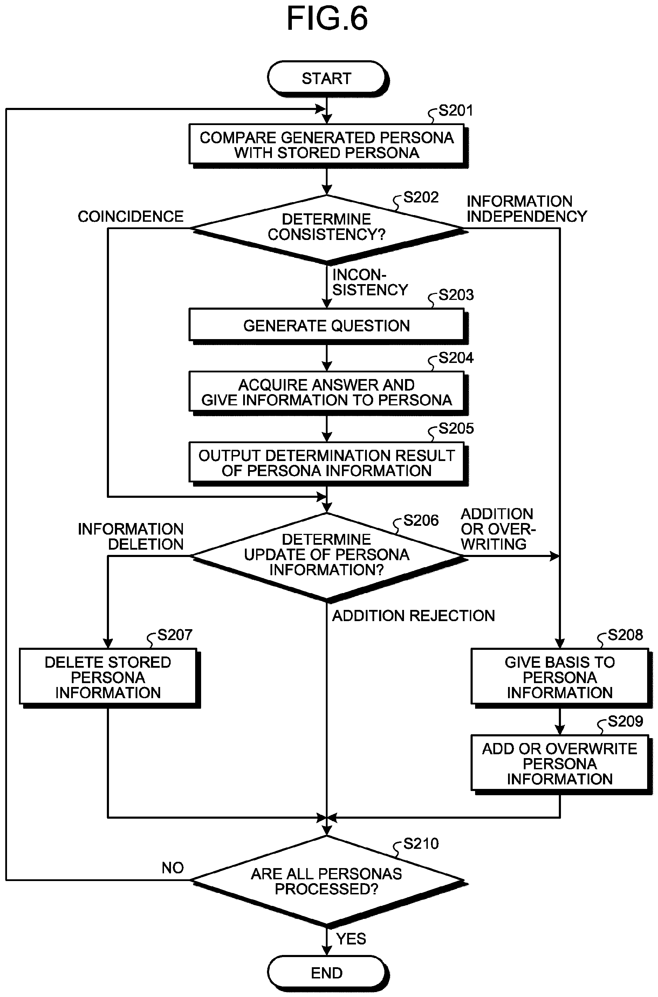

Next, a procedure of the determination processing according to the first embodiment is described with reference to FIGS. 5 and 6. First, generation processing of the first persona information is described with reference to FIG. 5. FIG. 5 is a flowchart (1) illustrating a flow of the determination processing according to the first embodiment.

As illustrated in FIG. 5, the determination device 100 receives an input of a text to be processed by means of the voice utterance and the like by the user 10 (step S101).

When the text received at step S101 is text Ti (0 < i <= N), the determination device 100 divides the text Ti into tj (0 < j <= n) (step S102). Note that, in a case where sentence division is not necessary, the determination device 100 skips step S102 (the above-described n is replaced with n = 1).

The determination device 100 generates a set of persona information (persona information group) for each divided text tj (step S103). The persona information generated at that time is persona information Px (0 < x <= M).

Subsequently, the determination device 100 gives a basis ey (0 < y <= K) to each persona information Px (step S104). Thereafter, the determination device 100 determines whether the basis is determined for all pieces of persona information (step S105). In a case where the bases of all pieces of persona information are not determined (step S105; No), that is, in a case where x < M, the determination device 100 gives a basis to next persona information.

In a case where the bases are given to all pieces of persona information (step S105; Yes), the determination device 100 stores the generated persona information group in the storage unit 120 (step S106). Note that, in a case where the determination device 100 does not give the basis to each persona information, this may skip steps S104 and S105.

Thereafter, the determination device 100 determines whether all the input texts are processed (step S107). In a case where not all the texts are processed (step S107; No), that is, in a case where i < N, the determination device 100 returns to step S103 and processes a next text while setting i = i + 1.

In contrast, in a case where all the texts are processed (step S107; Yes), that is, in a case where i = N, the determination device 100 finishes generating the persona information.

Next, a procedure of the determination processing according to the first embodiment is described with reference to FIG. 6. FIG. 6 is a flowchart (2) illustrating a flow of the determination processing according to the first embodiment.

The determination device 100 compares the generated first persona information with the stored second persona information (step S201). Similarly to FIG. 5, the persona information to be compared is Px (0 < x <= M).

The determination device 100 determines a relationship between the persona information Px and persona information p'y (0 < y <= L) that is one piece of the stored second persona information (step S202).

In a case where a result of the determination at step S202 is "inconsistent", the determination device 100 generates a question regarding inconsistent information (step S203). Specifically, the determination device 100 generates a question to confirm a difference between the first persona information Px and the second persona information p'y.

Then, the determination device 100 acquires an answer to the question, and gives information of the acquired answer to the persona information (step S204). Then, the determination device 100 outputs a determination result of the persona information (step S205). Note that, at that time point, the determination device 100 determines whether to update (store) or discard the persona information determined to be inconsistent at step S202.

In a case where the result of the determination at step S202 is "coincidence", the determination device 100 does not generate a question about the persona information, and advances the procedure to step S206.

The determination device 100 performs update determination of the persona information (step S206). For example, in a case where the second persona information does not indicate the current situation or in a case where the second persona information does not match the current situation, the determination device 100 deletes the stored persona information (step S207). Alternatively, in a case where the determination device 100 determines that the generated persona information is wrong or the stored persona information is more accurate, this discards the generated first persona information and advances the procedure.

In a case of determining that the pieces of information are independent at step S202 and in a case of determining the information addition or information overwriting at step S206, the determination device 100 gives the basis to new persona information (step S208). Then, the determination device 100 adds the persona information to the persona information storage unit 121 or overwrites the same (step S209).

Thereafter, the determination device 100 determines whether all the input pieces of persona information Px are processed (step S210). In a case where not all the pieces of persona information are processed (step S210; No), that is, in a case where x < M, the determination device 100 returns to step S201 and processes next persona information while setting x = x + 1.

In contrast, in a case where all the pieces of persona information are processed (step S210; Yes), that is, in a case where x = M, the determination device 100 finishes determining the persona information.

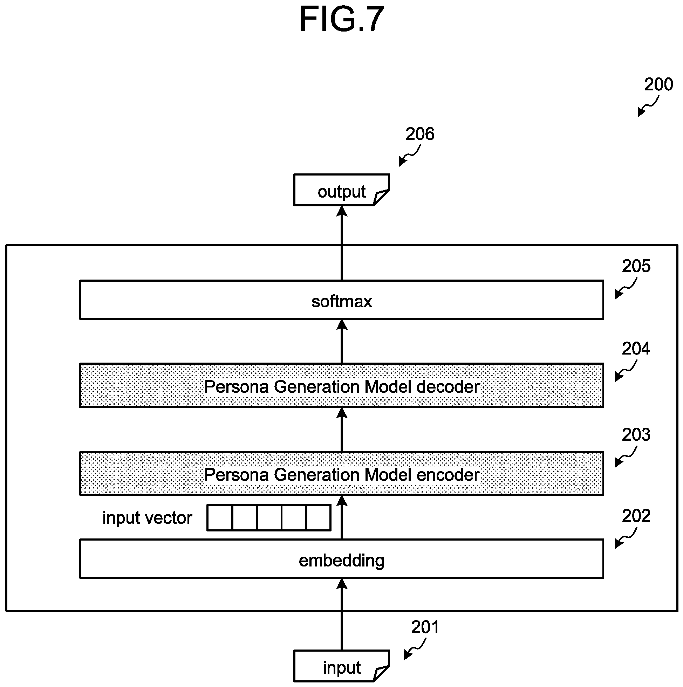

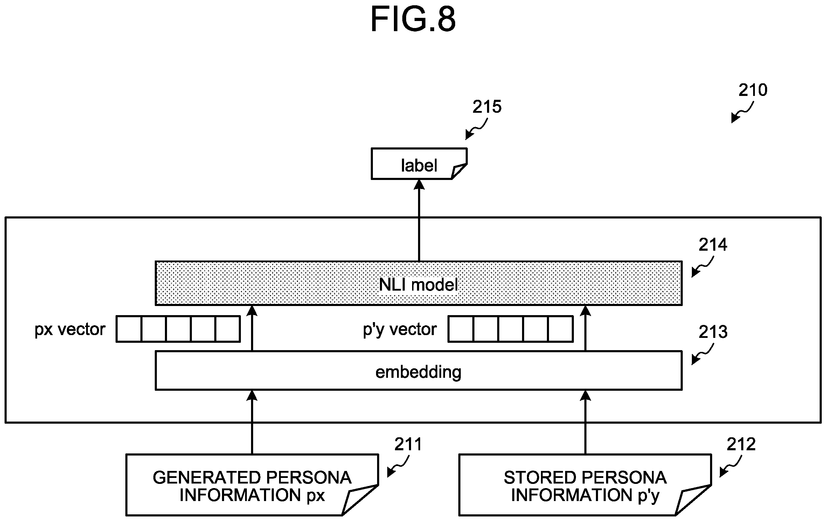

Herein, with reference to FIGS. 7 and 8, a model used for generation processing, determination processing and the like of persona information will be exemplified. FIG. 7 is an example of a conceptual diagram of a model configuration regarding the generation processing of the persona information.

The determination device 100 handles the received text as an input 201 of a model 200. In the model 200, the text is converted into a vector in an embedding layer 202, and the converted vector is processed by a persona information generation encoder 203 and a persona information generation decoder 204. Thereafter, the model 200 outputs persona information 206 through a softmax function 205.

FIG. 8 is an example of a conceptual diagram of a model configuration regarding the determination processing of the persona information. As illustrated in FIG. 8, the determination device 100 inputs generated persona information Px (first persona information) 211 and stored persona information p'y (second persona information) 212 to an embedding layer 213 of a model 210. In the model 210, a vector output from the embedding layer 213 is input to a natural language inference model 214, and a label 215 indicating whether the input pieces of information are inconsistent or information independency is output.

Note that, the model configuration described above is an example, and the determination device 100 may perform the generation processing and determination processing using various known models.

### (1-4. Specific Example of Determination Processing according to First Embodiment)

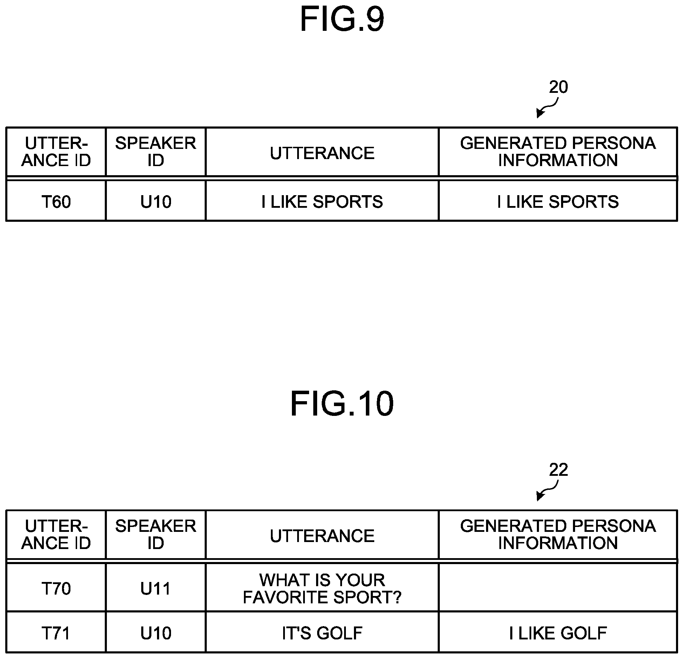

Next, a specific example of the determination processing according to the first embodiment is described with reference to FIG. 9 and subsequent drawings.

FIG. 9 is a diagram (1) illustrating a specific example of the determination processing according to the first embodiment. FIG. 9 illustrates a data table 20 in which the persona information is stored. In a case of receiving an input of utterance "I like sports" from the user 10, the determination device 100 generates persona information "I like sports" corresponding to such utterance.

FIG. 10 is a diagram (2) illustrating a specific example of the determination processing according to the first embodiment. FIG. 10 illustrates a data table 22 in which the persona information is stored. In an interaction between a user U11 and the user 10, the determination device 100 acquires, in response to a question of "What is your favorite sport?" of the user U11, utterance "It's golf" of the user 10. Then, the determination device 100 generates persona information "I like golf" corresponding to such utterance.

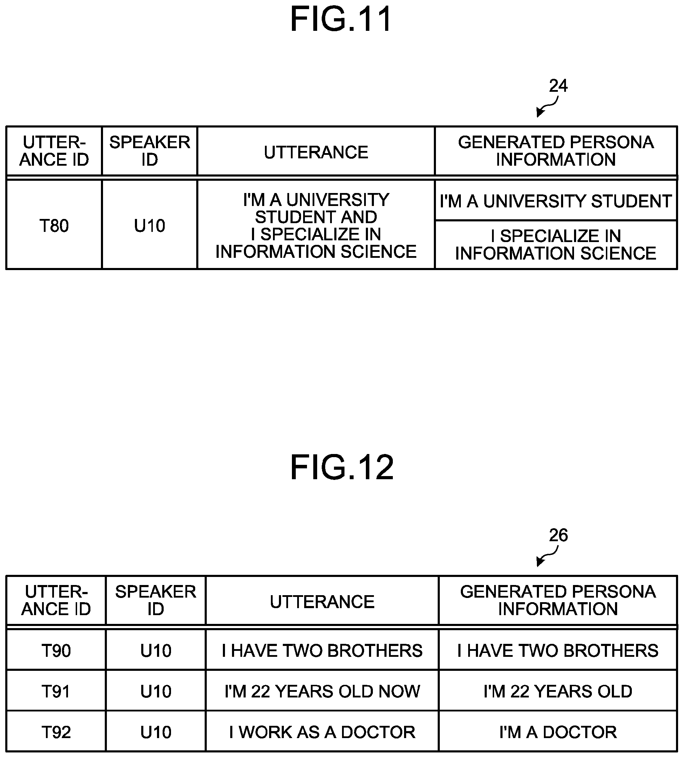

FIG. 11 is a diagram (3) illustrating a specific example of the determination processing according to the first embodiment. FIG. 11 illustrates a data table 24 in which the persona information is stored. The determination device 100 receives an input of utterance "I' m a university student and specialize in information science" from the user 10. In this case, the determination device 100 generates a plurality of pieces of persona information "I'm a university student" and "I specialize in information science" corresponding to such utterance.

FIG. 12 is a diagram (4) illustrating a specific example of the determination processing according to the first embodiment. FIG. 12 illustrates a data table 26 in which the persona information is stored. In a case of receiving an input of utterance "I have two brothers" from the user 10, the determination device 100 generates persona information "I have two brothers" corresponding to such utterance. In a case of receiving an input of utterance "I'm 22 years old now" from the user 10, the determination device 100 generates persona information "I'm 22 years old" corresponding to such utterance. In a case of receiving an input of utterance "I work as a doctor" from the user 10, the determination device 100 generates persona information "I'm a doctor" corresponding to such utterance.

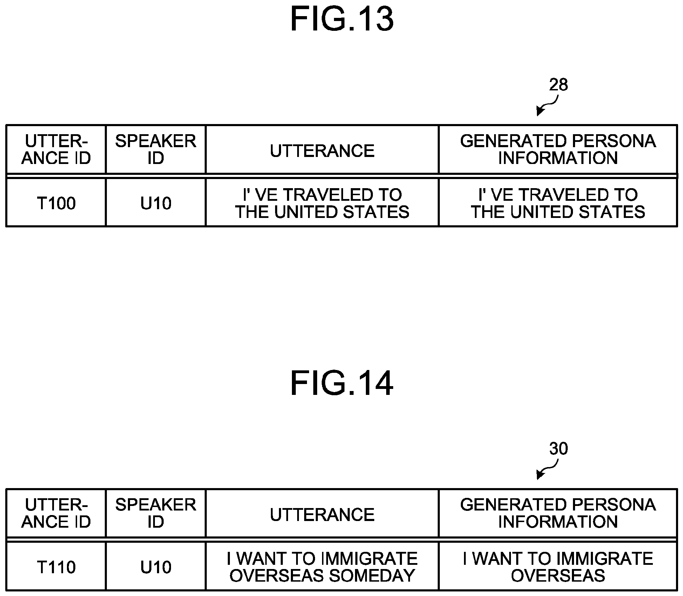

FIG. 13 is a diagram (5) illustrating a specific example of the determination processing according to the first embodiment. FIG. 13 illustrates a data table 28 in which the persona information is stored. In a case of receiving an input of utterance "I've traveled to the United States" from the user 10, the determination device 100 generates persona information "I've traveled to the United States" corresponding to such utterance.

FIG. 14 is a diagram (6) illustrating a specific example of the determination processing according to the first embodiment. FIG. 14 illustrates a data table 30 in which the persona information is stored. In a case of receiving an input of utterance "I want to immigrate overseas someday" from the user 10, the determination device 100 generates persona information "I want to immigrate overseas" corresponding to such utterance.

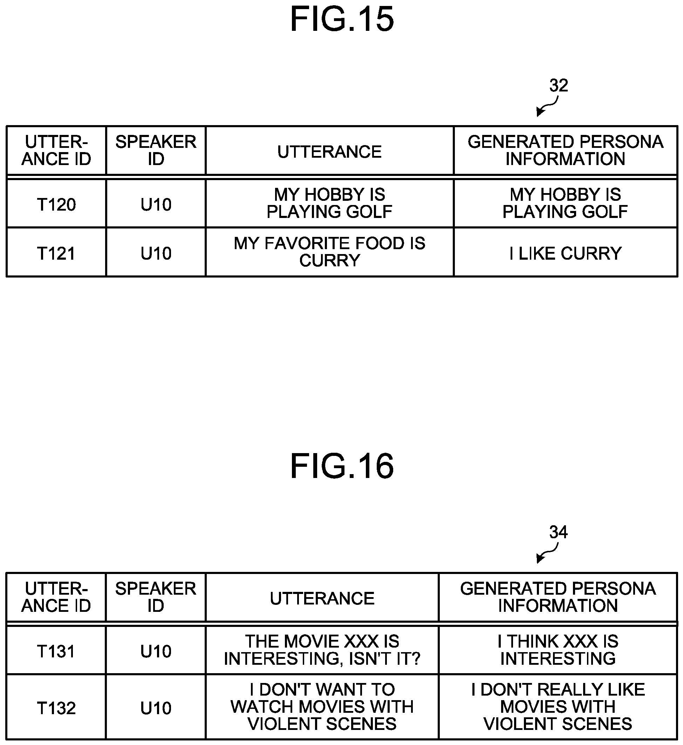

FIG. 15 is a diagram (7) illustrating a specific example of the determination processing according to the first embodiment. FIG. 15 illustrates a data table 32 in which the persona information is stored. In a case of receiving an input of utterance "My hobby is playing golf" from the user 10, the determination device 100 generates persona information "My hobby is playing golf" corresponding to such utterance. In a case of receiving an input of utterance "My favorite food is curry" from the user 10, the determination device 100 generates persona information "I like curry" corresponding to such utterance.

FIG. 16 is a diagram (8) illustrating a specific example of the determination processing according to the first embodiment. FIG. 16 illustrates a data table 34 in which the persona information is stored. In a case of receiving an input of utterance "The movie XXX is interesting, isn't it?" from the user 10, the determination device 100 generates persona information "I think XXX is interesting" corresponding to such utterance. In a case of receiving an input of utterance "I don't want to watch movies with violent scenes" from the user 10, the determination device 100 generates persona information "I don't really like movies with violent scenes" corresponding to such utterance. Note that, in the example illustrated in FIG. 16, the determination device 100 may generate the persona information on the basis of behavior information indicating that the user 10 has watched the movie entitled "XXX" or behavior information indicating that the user has stopped watching a violent movie in the middle instead of the utterance.

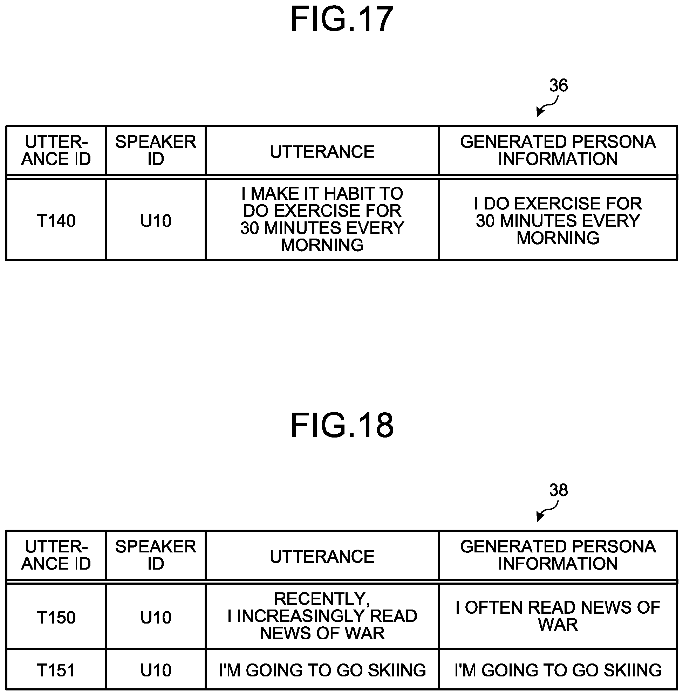

FIG. 17 is a diagram (9) illustrating a specific example of the determination processing according to the first embodiment. FIG. 17 illustrates a data table 36 in which the persona information is stored. In a case of receiving an input of utterance "I make it habit to do exercise for 30 minutes every morning" from the user 10, the determination device 100 generates persona information "I do exercise for 30 minutes every morning" corresponding to such utterance. Note that, in the example illustrated in FIG. 17, the determination device 100 may generate the persona information on the basis of a result of detecting that the user 10 does exercise for 30 minutes every morning and the like from the information stored in the wearable device of the user 10.

FIG. 18 is a diagram (10) illustrating a specific example of the determination processing according to the first embodiment. FIG. 18 illustrates a data table 38 in which the persona information is stored. In a case of receiving an input of utterance "Recently, I increasingly read news of war" from the user 10, the determination device 100 generates persona information "I often read news of war" corresponding to such utterance. In a case of receiving an input of utterance "I'm going to go skiing" from the user 10, the determination device 100 generates persona information "I'm going to go skiing" corresponding to such utterance.

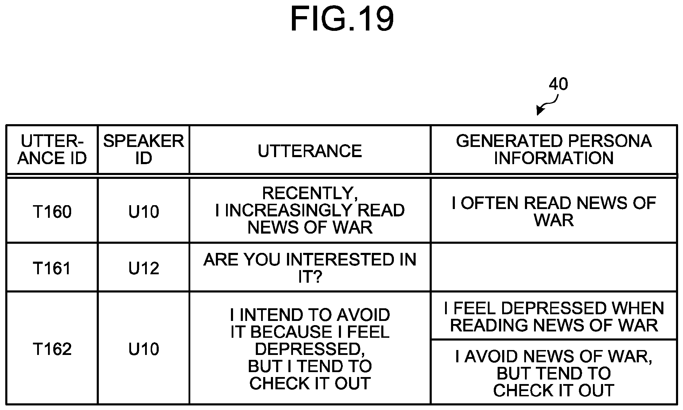

FIG. 19 is a diagram (11) illustrating a specific example of the determination processing according to the first embodiment. FIG. 19 illustrates a data table 40 in which the persona information is stored. In a case of receiving an input of utterance "Recently, I increasingly read news of war" from the user 10 from an interaction between a user U12 and the user 10, the determination device 100 generates persona information "I often read news of war" corresponding to such utterance. The determination device 100 receives an input of utterance "I intend to avoid it because I feel depressed, but I tend to check it out" uttered by the user 10 in response to a subsequent question from the user U12. In this case, the determination device 100 generates a plurality of pieces of persona information "I feel depressed when reading news of war" and "I avoid news of war, but tend to check it out" corresponding to such utterance.

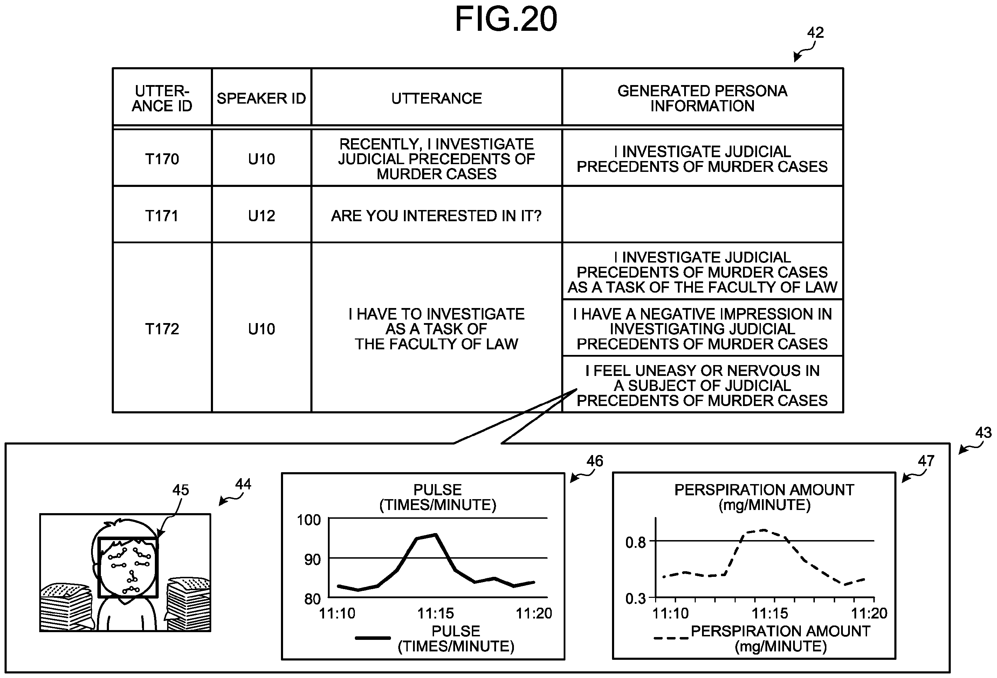

FIG. 20 is a diagram (12) illustrating a specific example of the determination processing according to the first embodiment. FIG. 20 illustrates a data table 42 in which the persona information is stored. In a case of receiving an input of utterance "Recently, I investigate judicial precedents of murder cases" of the user 10 from the interaction between the user U12 and the user 10, the determination device 100 generates persona information "I investigate judicial precedents of murder cases" corresponding to such utterance.

The determination device 100 receives an input of an utterance "I have to investigate as a task of the faculty of law" uttered by the user 10 in response to a subsequent question from the user U12. At that time, the determination device 100 acquires additional information 43 together with the utterance of the user 10. For example, the determination device 100 acquires image information 44, pulse information 46, and perspiration amount information 47. The image information 44 includes feature information 45 for determining the expression of the user.

The determination device 100 generates persona information "I investigate judicial precedents of murder cases as a task of the faculty of law" corresponding to such utterance. The determination device 100 scores that the user is not interested in the investigation, that is, the user has a negative expression, from the expression of the user. The determination device 100 generates persona information "I have a negative impression in investigating judicial precedents of murder cases" corresponding to such expression. The determination device 100 scores a degree to which the pulse or the perspiration amount of the user 10 deviate from a normal value together with the utterance of the user 10. The determination device 100 generates persona information "I feel uneasy or nervous in a subject of judicial precedents of murder cases" corresponding to such trend in biological information.

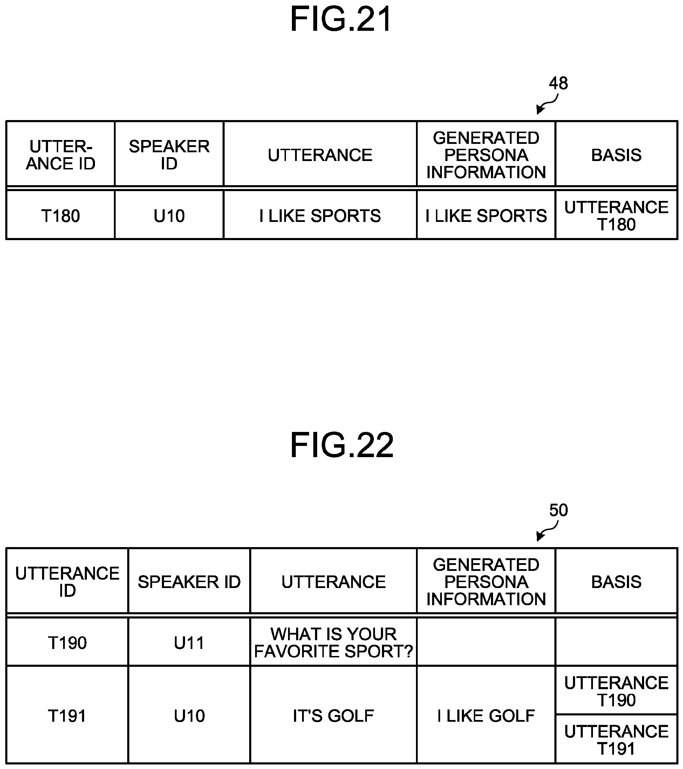

Next, an example of giving the basis to the persona information out of specific examples of the determination processing according to the first embodiment is described with reference to FIG. 21 and subsequent drawings.

FIG. 21 is a diagram (13) illustrating a specific example of the determination processing according to the first embodiment. FIG. 21 illustrates a data table 48 in which the persona information is stored. In a case of receiving an input of utterance "I like sports" from the user 10, the determination device 100 generates persona information "I like sports" corresponding to such utterance. The determination device 100 gives "utterance T180" that is information for identifying the utterance as the basis for generating the persona information.

FIG. 22 is a diagram (14) illustrating a specific example of the determination processing according to the first embodiment. FIG. 22 illustrates a data table 50 in which the persona information is stored. In the interaction between the user U11 and the user 10, the determination device 100 acquires, in response to a question of "What is your favorite sport?" of the user U11, utterance "It's golf" of the user 10. Then, the determination device 100 generates persona information "I like golf" corresponding to such utterance. The determination device 100 gives "utterance T190" and "utterance T191" that are pieces of information for identifying the utterance as the bases for generating the persona information.

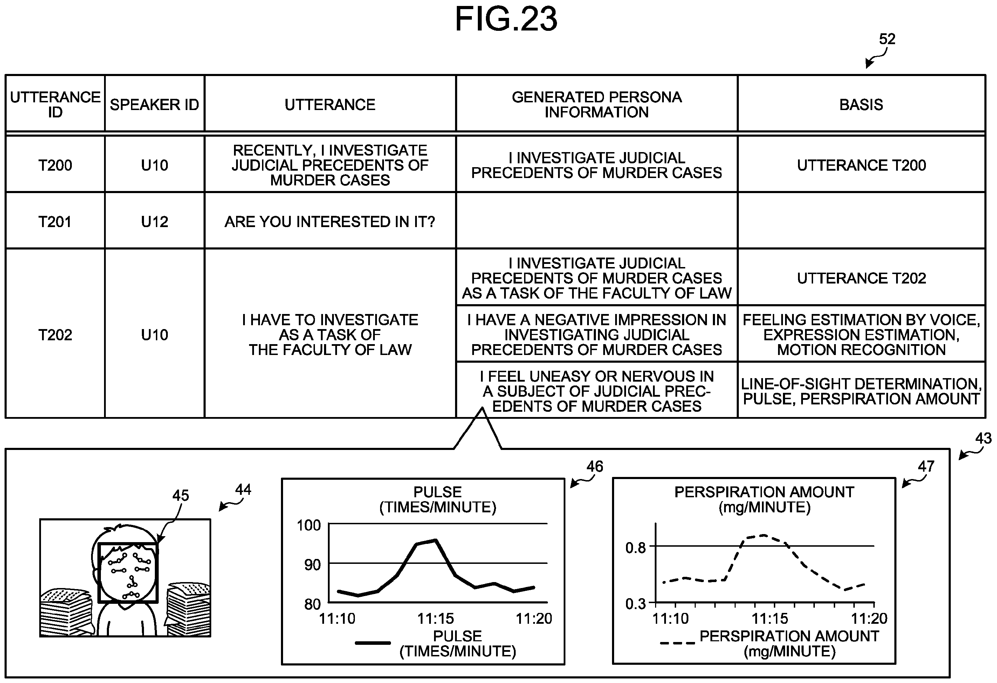

FIG. 23 is a diagram (15) illustrating a specific example of the determination processing according to the first embodiment. FIG. 23 illustrates a data table 52 in which the persona information is stored. In a case of receiving an input of utterance "Recently, I investigate judicial precedents of murder cases" of the user 10 from the interaction between the user U12 and the user 10, the determination device 100 generates persona information "I investigate judicial precedents of murder cases" corresponding to such utterance.

The determination device 100 receives an input of an utterance "I have to investigate as a task of the faculty of law" uttered by the user 10 in response to a subsequent question from the user U12. At that time, the determination device 100 acquires additional information 43 together with the utterance of the user 10 similar to the example illustrated in FIG. 17. For example, the determination device 100 acquires the image information 44, pulse information 46, and perspiration amount information 47. The image information 44 includes the feature information 45 for determining the expression of the user.

The determination device 100 generates persona information "I investigate judicial precedents of murder cases as a task of the faculty of law" corresponding to such utterance. The determination device 100 gives "utterance T200" that is information for identifying the utterance as the basis for generating the persona information.

The determination device 100 scores that the user is not interested in the investigation, that is, the user has a negative expression, from the expression of the user. The determination device 100 generates persona information "I have a negative impression in investigating judicial precedents of murder cases" corresponding to such expression. The determination device 100 gives feeling estimation by voice, expression estimation in the feature information 45 included in the image information 44, the motion of the user 10 in the image information 44 and the like as the bases for generating the persona information.

The determination device 100 scores a degree to which the pulse or the perspiration amount of the user 10 deviate from a normal value together with the utterance of the user 10. The determination device 100 generates persona information "I feel uneasy or nervous in a subject of judicial precedents of murder cases" corresponding to such trend in biological information. The determination device 100 gives line-of-sight determination such as that the line of sight of the user 10 is not stable, a numerical value in the pulse information 46 and the perspiration amount information 47 and the like as the bases for generating the persona information.

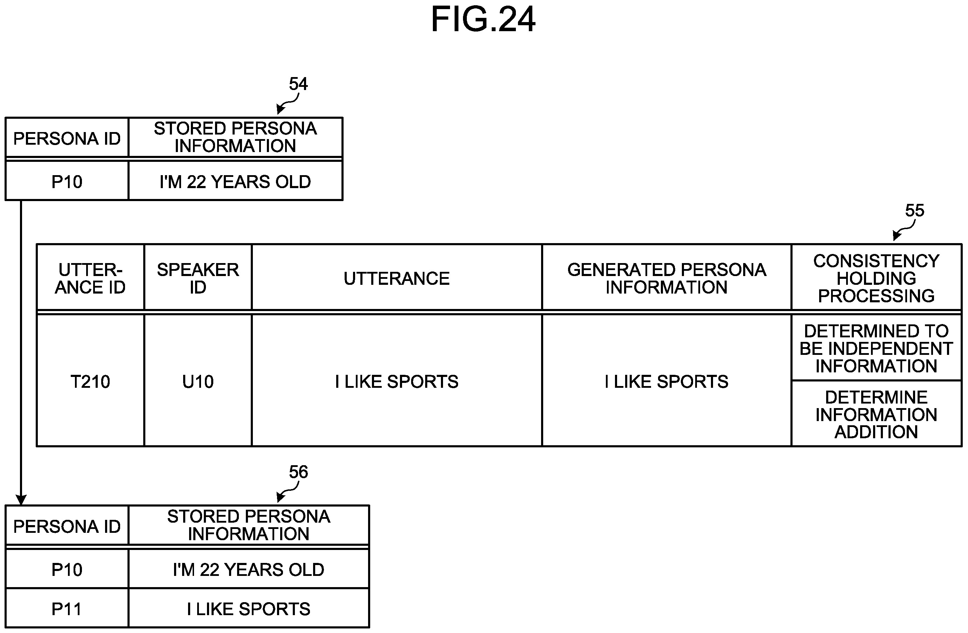

Next, an example of a case of determining consistency out of the specific examples of the determination processing according to the first embodiment is described with reference to FIG. 24 and subsequent drawings.

FIG. 24 is a drawing (16) illustrating a specific example of the determination processing according to the first embodiment. FIG. 24 illustrates a data table 54 in which the second persona information is stored. In a case of receiving an input of utterance "I like sports" from the user 10, the determination device 100 generates persona information "I like sports" corresponding to such utterance. The determination device 100 compares the persona information with the persona information held in the data table 54, and determines that they are pieces of independent information because the attribute and the target are different. Then, the determination device 100 determines to add newly generated persona information as illustrated in a processing table 55.

In this case, as illustrated in a data table 56, the determination device 100 holds newly generated persona information P11 together with originally held persona information P10.

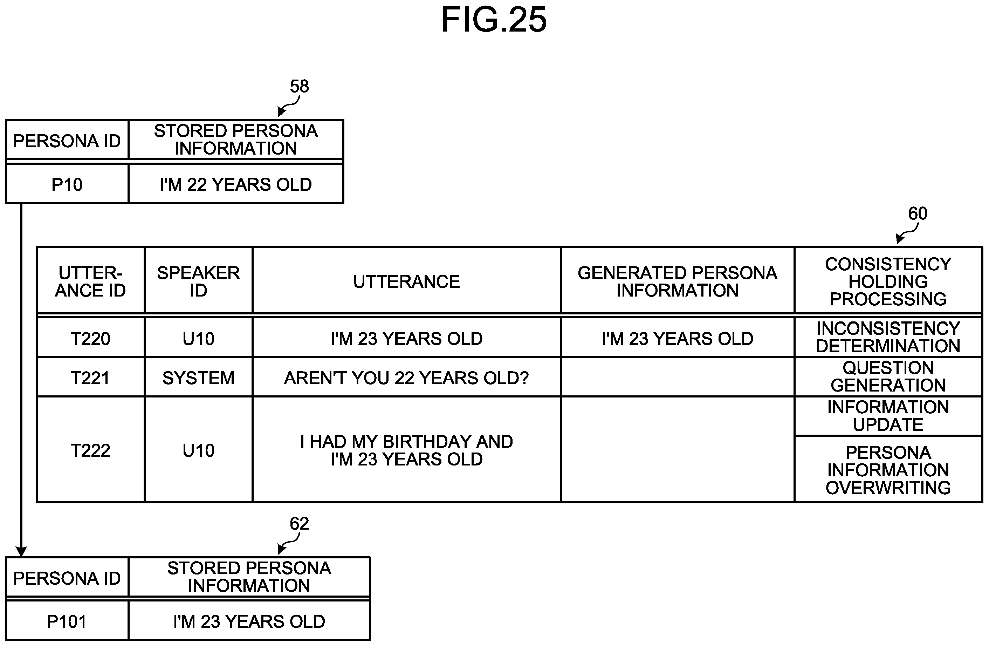

FIG. 25 is a diagram (17) illustrating a specific example of the determination processing according to the first embodiment. FIG. 25 illustrates a data table 58 in which the second persona information is stored. In a case of receiving an input of utterance "I'm 23 years old" from the user 10, the determination device 100 generates persona information "I'm 23 years old" corresponding to such utterance. The determination device 100 compares the persona information with the persona information held in the data table 58, and determines that they are inconsistent because the attribute and the target are the same but the indicated information is different.

In this case, the determination device 100 generates a question corresponding to the information determined to be inconsistent. Specifically, in order to ask a question about the age of the user 10, the determination device 100 generates a question such as "Aren't you 22 years old?" and outputs the question to the user 10.

Thereafter, when obtaining information "I had my birthday and I'm 23 years old" as an answer of the user 10, the determination device 100 determines to update the persona information on the basis of such information. In this case, since the age of the user 10 is uniquely determined information, the determination device 100 determines to overwrite overlapping persona information P10 as illustrated in a processing table 60.

Then, as illustrated in a data table 62, the determination device 100 overwrites the originally held persona information P10 with persona information P101 and updates the persona information of the user 10.

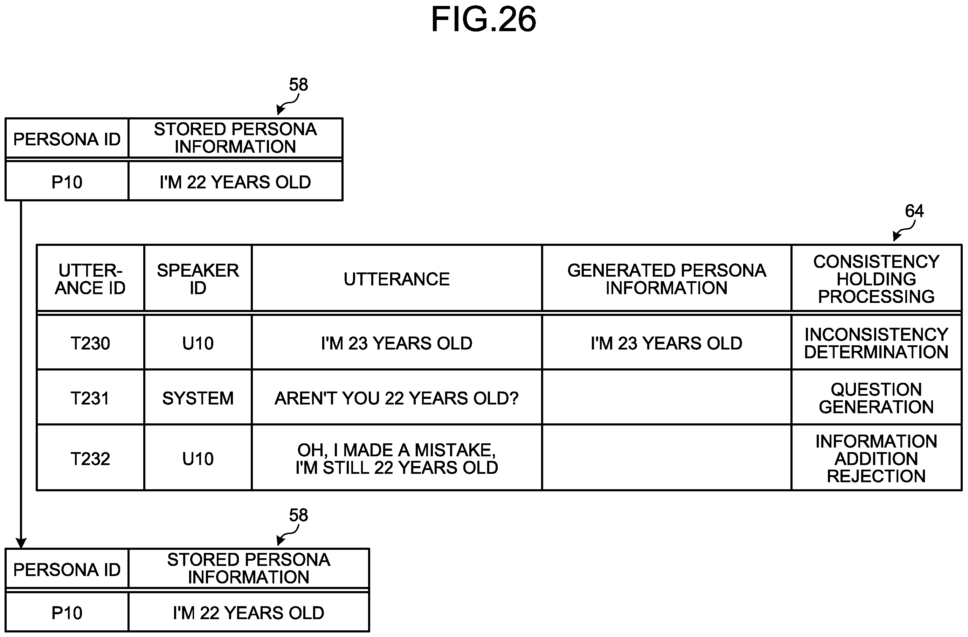

FIG. 26 is a diagram (18) illustrating a specific example of the determination processing according to the first embodiment. FIG. 26 illustrates a data table 58 in which the second persona information is stored. In a case of receiving an input of utterance "I'm 23 years old" from the user 10, the determination device 100 generates persona information "I'm 23 years old" corresponding to such utterance. The determination device 100 compares the persona information with the persona information held in the data table 58, and determines that they are inconsistent because the attribute and the target are the same but the indicated information is different.

In this case, the determination device 100 generates a question corresponding to the information determined to be inconsistent. Specifically, in order to ask a question about the age of the user 10, the determination device 100 generates a question such as "Aren't you 22 years old?" and outputs the question to the user 10.

Thereafter, when obtaining information "Oh, I made a mistake, I'm still 22 years old" as the answer of the user 10, the determination device 100 determines that the information indicated by the newly generated persona information is incorrect on the basis of the information. In this case, since the age of the user 10 is uniquely determined information, the determination device 100 determines to discard the newly generated persona information as illustrated in a processing table 64.

Then, as illustrated in the data table 58, the determination device 100 maintains the originally held persona information P10.

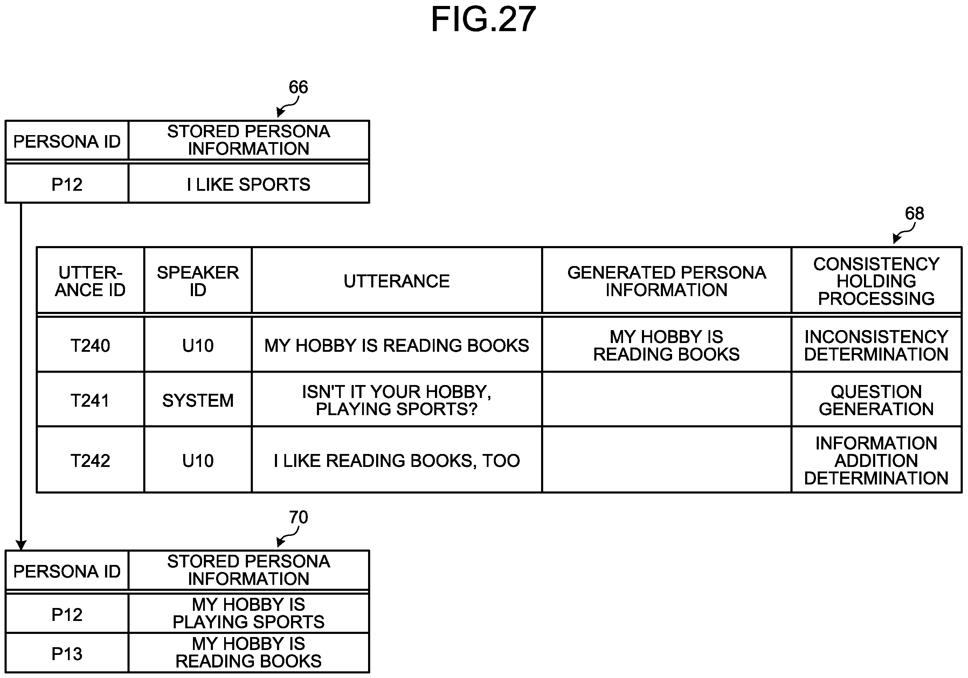

FIG. 27 is a diagram (19) illustrating a specific example of the determination processing according to the first embodiment. FIG. 27 illustrates a data table 66 in which the second persona information is stored. In a case of receiving an input of utterance "My hobby is reading books" from the user 10, the determination device 100 generates persona information "My hobby is reading books" corresponding to such utterance. The determination device 100 compares the persona information with the persona information held in the data table 66, and determines that they are inconsistent because the attribute and the target are the same but the indicated information is different.

In this case, the determination device 100 generates a question corresponding to the information determined to be inconsistent. Specifically, in order to confirm the hobby of the user 10, the determination device 100 generates a question such as "Isn't it your hobby, playing sports?" and outputs the question to the user 10.

Thereafter, when obtaining information "I like reading books, too" as an answer of the user 10, the determination device 100 determines that the newly generated persona information is information that should be added on the basis of such information. That is, since there may a plurality of hobbies of the user 10 without problem, the determination device 100 determines to add the newly generated persona information as illustrated in a processing table 68.

Then, as illustrated in a data table 70, the determination device 100 holds persona information P13 in addition to the originally held persona information P12.

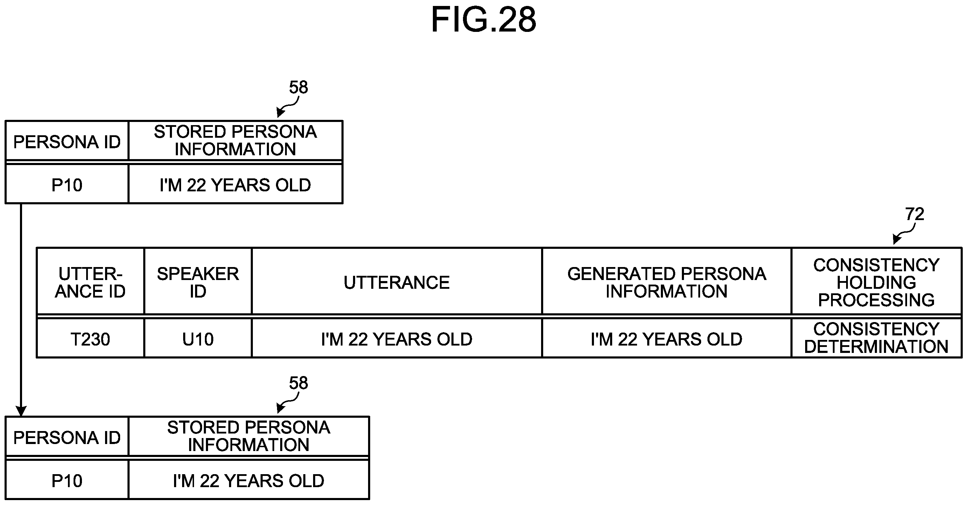

FIG. 28 is a diagram (20) illustrating a specific example of the determination processing according to the first embodiment. FIG. 28 illustrates the data table 58 in which the second persona information is stored. In a case of receiving an input of utterance "I'm 22 years old" from the user 10, the determination device 100 generates persona information "I'm 22 years old" corresponding to such utterance. The determination device 100 compares the persona information with the persona information held in the data table 58, and determines that they coincide because the attribute and the target are the same and the indicated information is also the same.

In this case, the determination device 100 determines that the persona information is consistent, and maintains the information in the data table 58.

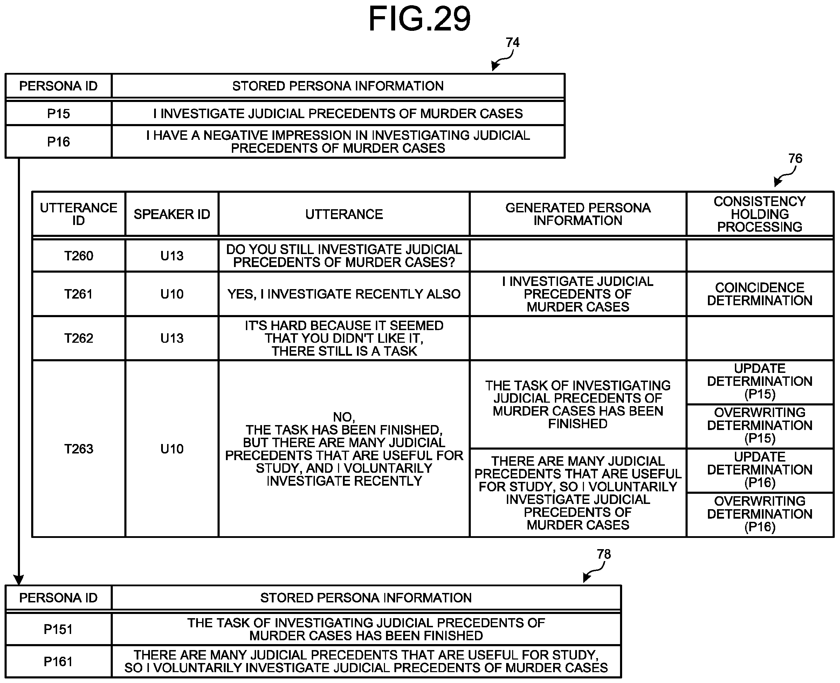

FIG. 29 is a diagram (21) illustrating a specific example of the determination processing according to the first embodiment. FIG. 29 illustrates a data table 74 in which the second persona information is stored. In a case of receiving an input of utterance "Yes, I investigate recently also" of the user 10 who answers a question of a user U13 in an interaction between the user U13 and the user 10, the determination device 100 generates persona information "I investigate judicial precedents of murder cases" corresponding to such utterance. The determination device 100 compares the persona information with the persona information held in the data table 74, and determines that they coincide because the attribute and the target are the same and the indicated information is also the same.

The determination device 100 receives, in response to an interaction by the user U13 "It's hard because it seemed that you didn't like it, there still is a task.", an input of an answer "No, the task has been finished, but there are many judicial precedents that are useful for study, and I voluntarily investigate recently" of the user 10. From such utterance, the determination device 100 generates persona information "The task of investigating judicial precedents of murder cases has been finished" and "There are many judicial precedents that are useful for study, so I voluntarily investigate judicial precedents of murder cases".

The determination device 100 determines that the information has been updated from persona information P15 held in the data table 74 regarding the persona information "The task of investigating judicial precedents of murder cases has been finished", and determines overwriting of the information.

The determination device 100 determines that the information has been updated from persona information P16 held in the data table 74 regarding the persona information "There are many judicial precedents that are useful for study, so I voluntarily investigate judicial precedents of murder cases" and determines overwriting of the information.

As a result, the determination device 100 holds persona information P151 and persona information P161 updated from the persona information P15 and the persona information P16 as illustrated in a data table 78.

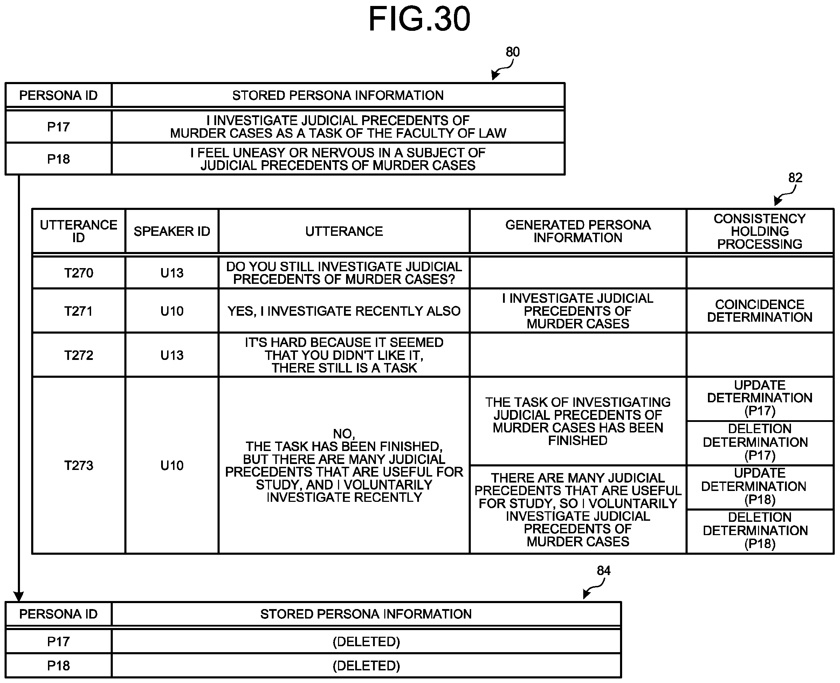

FIG. 30 is a drawing (22) illustrating a specific example of the determination processing according to the first embodiment. FIG. 30 illustrates a data table 80 in which the second persona information is stored. In a case of receiving an input of utterance "Yes, I investigate recently also" of the user 10 who answers the question of the user U13 in the interaction between the user U13 and the user 10, the determination device 100 generates persona information "I investigate judicial precedents of murder cases" corresponding to such utterance. The determination device 100 compares the persona information with the persona information held in the data table 80, and determines that they coincide because the attribute and the target are the same and the indicated information is also the same.

The determination device 100 receives, in response to an interaction by the user U13 "It's hard because it seemed that you didn't like it, there still is a task.", an input of an answer "No, the task has been finished, but there are many judicial precedents that are useful for study, and I voluntarily investigate recently" of the user 10. From such utterance, the determination device 100 generates persona information "The task of investigating judicial precedents of murder cases has been finished" and "There are many judicial precedents that are useful for study, so I voluntarily investigate judicial precedents of murder cases".

The determination device 100 determines that the information has been deleted from persona information P17 held in the data table 80 as for the persona information "The task of investigating judicial precedents of murder cases has been finished", and determines to delete the original persona information P17.

The determination device 100 determines that the information has been updated from persona information P18 held in the data table 80 regarding the persona information "There are many judicial precedents that are useful for study, so I voluntarily investigate judicial precedents of murder cases" and determines to delete the original persona information P18.

As a result, as illustrated in a data table 84, the determination device 100 deletes the persona information P17 and persona information P18. Note that, the determination device 100 may hold a history of deletion of the persona information P17 and persona information P18, and handle such deletion history as persona information indicating the past behavior of the user 10.

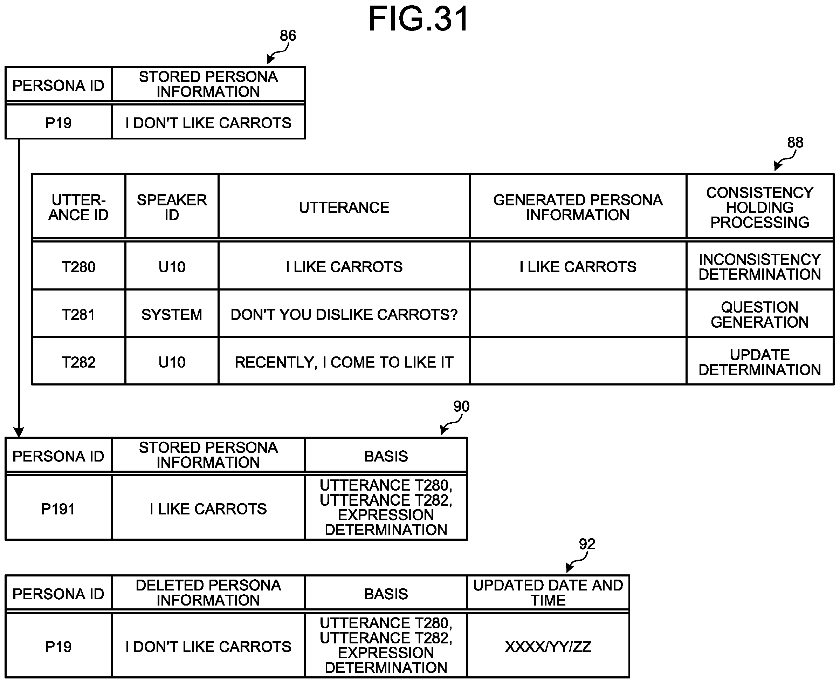

FIG. 31 is a diagram (23) illustrating a specific example of the determination processing according to the first embodiment. FIG. 31 illustrates a data table 86 in which the second persona information is stored. In a case of receiving an input of utterance "I like carrots" of the user 10 from the interaction with the user 10, the determination device 100 generates persona information "I like carrots" corresponding to such utterance. The determination device 100 compares the persona information with the persona information held in the data table 86, and determines that they are inconsistent because the attribute and the target are the same but the indicated information is different.

In this case, the determination device 100 generates a question corresponding to the information determined to be inconsistent. Specifically, in order to ask a question about the taste of the user 10, the determination device 100 generates a question such as "Don't you dislike carrots?" and outputs the question to the user 10.

Thereafter, in response to utterance "Recently, I come to like it" by user 10, the determination device 100 determines that past persona information P19 has been updated on the basis of such utterance.

In this case, as illustrated in a data table 90, the determination device 100 holds newly generated persona information P191 and gives utterance T280 and utterance T282, which is information as the basis when generating and updating the persona information P191. At that time, the determination device 100 may give, as the basis, that the score has been high, such as that the user 10 had a smiling face in expression determination of the user 10 at the time of the utterance T280.

Moreover, as illustrated in a data table 92, the determination device 100 may hold a history of the deleted persona information P19. In this case, the determination device 100 may give the basis for updating the information, and may hold date and time information indicating when the information of user 10 changes.

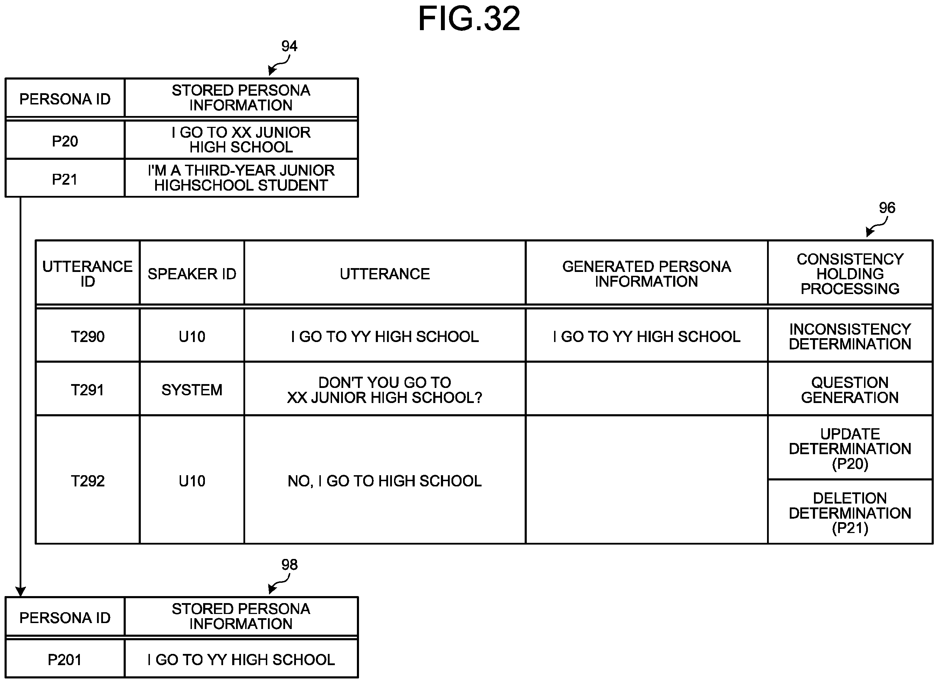

FIG. 32 is a diagram (24) illustrating a specific example of the determination processing according to the first embodiment. FIG. 32 illustrates a data table 94 in which the second persona information is stored. In a case of receiving an input of utterance "I go to YY high school" of the user 10 from the interaction with the user 10, the determination device 100 generates persona information "I go to YY high school" corresponding to such utterance. The determination device 100 compares the persona information with the persona information held in the data table 94, and determines that they are inconsistent because the attribute and the target are the same but the indicated information is different.

In this case, the determination device 100 generates a question corresponding to the information determined to be inconsistent. Specifically, in order to ask a question about the situation of the user 10, the determination device 100 generates a question such as "Don't you go to XX junior high school?" and outputs the question to the user 10.

Thereafter, in response to utterance "No, I go to high school now" by the user 10, the determination device 100 determines that past persona information P20 and persona information P21 have been updated on the basis of such utterance.

In this case, as illustrated in a data table 98, the determination device 100 holds newly generated persona information P201 and deletes persona information P20 and persona information P21, which indicate information inconsistent with the persona information P201.

### (2. Second Embodiment)

Next, a second embodiment is described. In the second embodiment, a determination device 100 generates persona information using information held in an external knowledge database. That is, a generation unit 132 according to the second embodiment generates first persona information on the basis of reaction information and information held in a predetermined knowledge database.

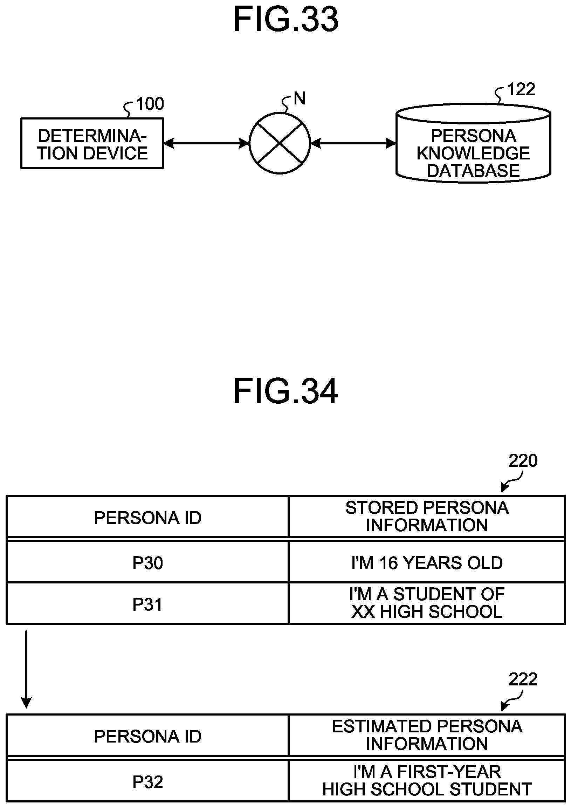

FIG. 33 is a diagram illustrating a configuration example of the determination device 100 according to the second embodiment. As illustrated in FIG. 33, the determination device 100 is connected to a persona knowledge database 122 via a network N. Then, the determination device 100 infers persona information that can be explicitly derived from a plurality of facts included in knowledge data stored in the persona knowledge database 122 and the persona information held or generated by the device itself.

A specific example of the second embodiment is described below with reference to FIG. 34 and subsequent drawings. FIG. 34 is a diagram (1) illustrating a specific example of determination processing according to the second embodiment.

In the example illustrated in FIG. 34, the determination device 100 includes a data table 220. In the data table 220, two pieces of persona information **"I'm 16** years old" and **"I'm** a student of XX high school" are held regarding a user 10.

At that time, the determination device 100 refers to the persona knowledge database 122, and obtains knowledge that a high school student who is 16 years old generally corresponds to "first-year high school student". Then, the determination device 100 logically infers information "I'm a first-year high school student" as the persona information of the user 10 on the basis of the persona information held in the data table 220. The determination device 100 stores inferred persona information P32 in a data table 222. Note that, the determination device 100 may add the inferred persona information P32 in the data table 220.

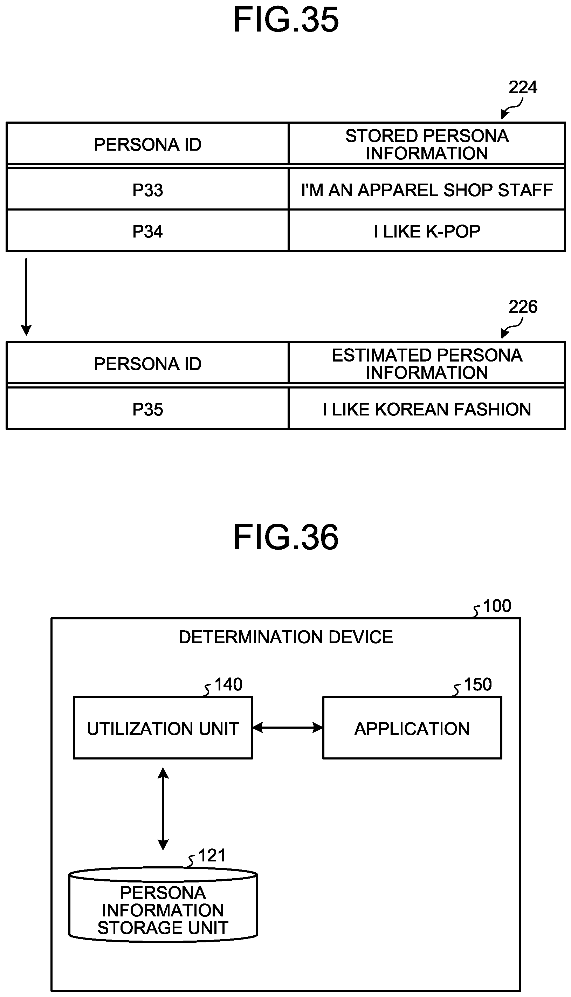

FIG. 35 is a diagram (2) illustrating a specific example of determination processing according to the second embodiment. In the example illustrated in FIG. 35, the determination device 100 includes a data table 224. In the data table 224, two pieces of persona information "I'm an apparel shop staff" and "I like K-POP" are held regarding the user 10.

At that time, the determination device 100 infers the persona information supposed from a plurality of attributes, hobbies, tastes and the like. In the example of FIG. 35, the determination device 100 derives information "I'm interested in fashion" from the persona information "I'm an apparel shop staff". The determination device 100 derives information "I like Korea" from the persona information "I like K-POP". Then, the determination device 100 logically infers persona information "I like Korean fashion" from the plurality of pieces of information.

Note that, in the case as illustrated in FIG. 35, it is supposed that an output inference result is different according to the knowledge database and the like referred to by the determination device 100. The determination device 100 may perform such inference processing using rule processing, statistical probability, a machine learning model and the like. As an example, the determination device 100 may infer by a method using a common knowledge graph.

The determination device 100 may determine consistency of the persona information before adding the inferred persona information to the persona information storage unit 121. According to the result, the determination device 100 may overwrite or delete the persona information stored in the persona information storage unit 121 in addition to adding the inferred persona information.

### (3. Third Embodiment)

Next, a third embodiment is described. In the third embodiment, a determination device 100 utilizes held persona information in another application.

FIG. 36 is a diagram illustrating a configuration example of the determination device 100 according to the third embodiment. As illustrated in FIG. 36, the determination device 100 includes a utilization unit 140 and an application 150.

The utilization unit 140 provides persona information regarding a user updated by an update unit 134 to a predetermined external program (application 150) to utilize. Note that, the application 150 may be executed by the determination device 100 or may be a program executed by a device different from the determination device 100.

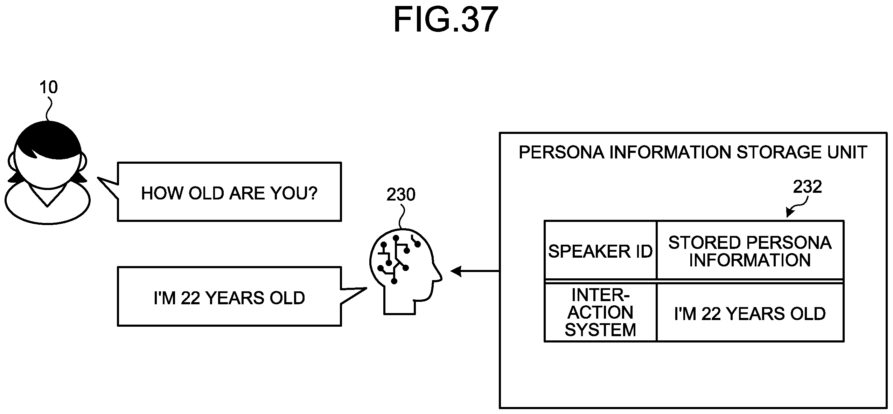

A specific example of the third embodiment is described with reference to FIG. 37 and subsequent drawings. FIG. 37 is a diagram (1) illustrating a specific example of determination processing according to the third embodiment. In the example of FIG. 37, the determination device 100 utilizes the persona information in an interaction system 230, which is an automatic interaction application such as voice chat or a communication support system.

In the example of FIG. 37, the determination device 100 holds the persona information of the interaction system 230 in a data table 232. In this case, when a user 10 asks the interaction system 230 "How old are you?", the interaction system 230 refers to the data table 232. Then, the interaction system 230 determines that persona information "I'm 22 years old" is held as its own attribute information. Then, the interaction system 230 outputs an answer "I'm 22 years old" to the user 10 on the basis of such persona information.

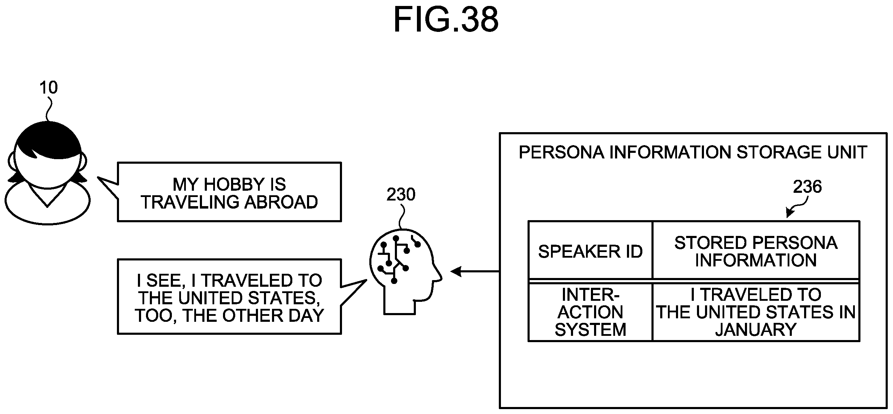

FIG. 38 is a diagram (2) illustrating a specific example of the determination processing according to the third embodiment. In the example of FIG. 38, the determination device 100 holds the persona information of the interaction system 230 in a data table 236. In this case, when the user 10 utters "My hobby is traveling abroad" to the interaction system 230, the interaction system 230 refers to the data table 236. Then, the interaction system 230 extracts persona information "I traveled to the United States in January", which is persona information having a high affinity with the utterance of the user 10 immediately before. Then, on the basis of such persona information, the interaction system 230 outputs utterance having a high affinity for the utterance of the user 10 "I see, I traveled to the United States, too, the other day".

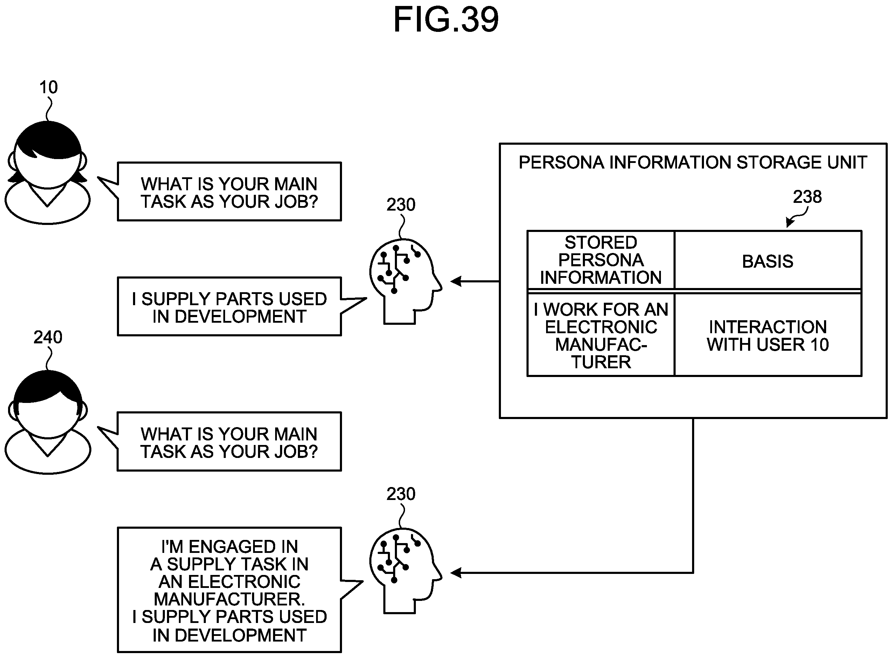

FIG. 39 is a diagram (3) illustrating a specific example of the determination processing according to the third embodiment. In the example of FIG. 39, the determination device 100 holds the persona information of the interaction system 230 in a data table 238. The persona information held in the data table 238 stores persona information "I work for an electronic manufacturer" and information that the persona information is generated by "interaction with the user 10". In this case, it is obvious for the user 10 that a character supposed by the interaction system 230 "works for an electronic manufacturer". In contrast, this means that users other than the user 10 do not know that the character supposed by the interaction system 230 "works for an electronic manufacturer".

In an example of FIG. 39, when the user 10 asks the interaction system 230 "What is your main task as your job?", the interaction system 230 refers to the data table 238. Then, since the persona information "I work for an electronic manufacturer" is obvious to the user 10, the interaction system 230 omits an output of such information and outputs other information as utterance.

In contrast, when a user 240 asks the interaction system 230 "What is your main task as your job?", the interaction system 230 refers to the data table 238. Since the persona information "I work for an electronic manufacturer" is not obvious to the user 240, the interaction system 230 outputs information "I'm engaged in a supply task in an electronic manufacturer.", which is not obvious to the user 240, on the assumption of such situation.

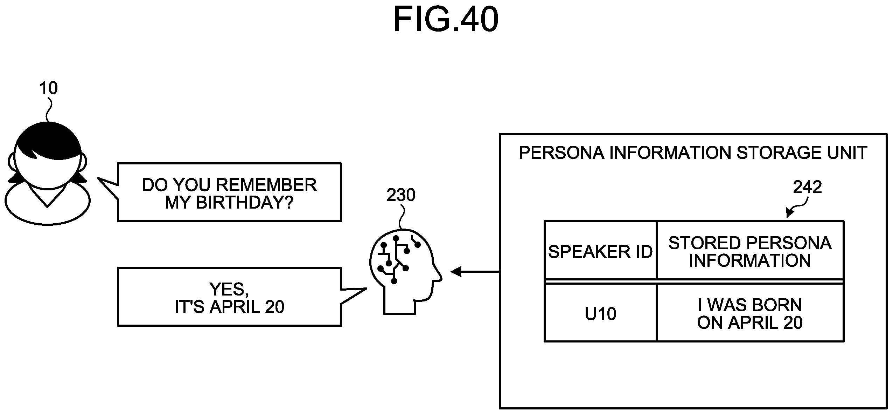

FIG. 40 is a diagram (4) illustrating a specific example of determination processing according to the third embodiment. In the example of FIG. 40, the determination device 100 holds persona information "I was born on April 20" associated with the user 10 in a data table 242.

In this case, when the user 10 asks the interaction system 230 "Do you remember my birthday?", the interaction system 230 refers to the data table 242. Then, the interaction system 230 refers to the persona information including the attribute information of the user 10, and outputs an appropriate answer such as "Yes, it's April 20" to the user 10 on the basis of such persona information.

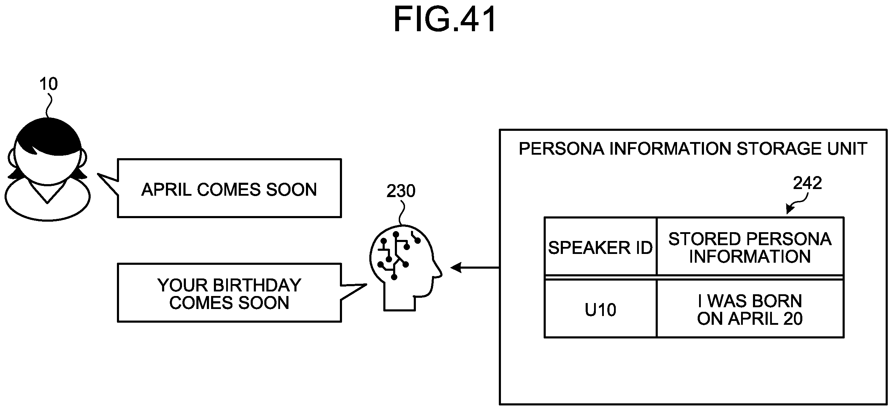

FIG. 41 is a diagram (5) illustrating a specific example of determination processing according to the third embodiment. In the example of FIG. 41, the determination device 100 holds persona information "I was born on April 20" associated with the user 10 in the data table 242.

In this case, when the user 10 utters "April comes soon" to the interaction system 230, the interaction system 230 refers to the data table 242. Then, the interaction system 230 refers to the persona information on the basis of a period of a topic talked about and the like, and outputs an answer personalized to the user 10 following a flow of the interaction such as "Your birthday comes soon" on the basis of such persona information.

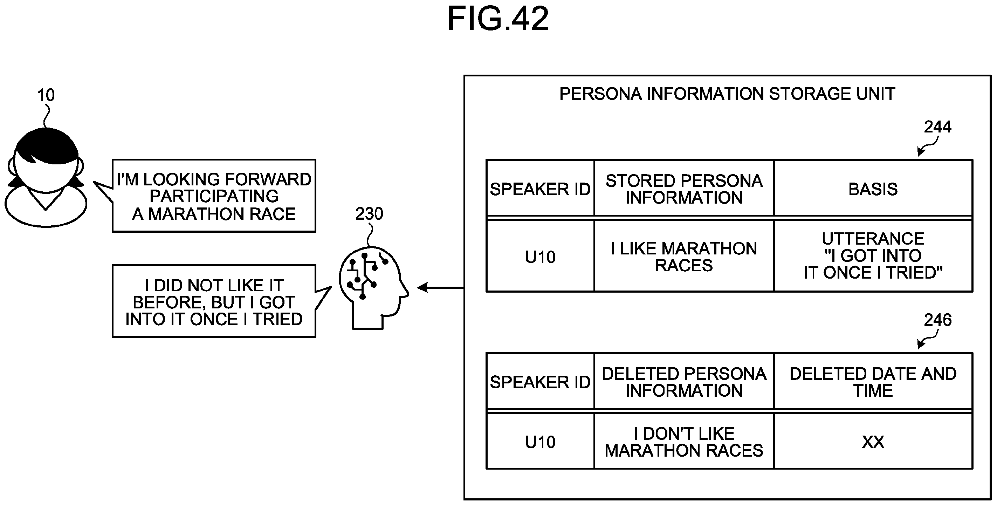

FIG. 42 is a diagram (6) illustrating a specific example of determination processing according to the third embodiment. In the example of FIG. 42, the determination device 100 holds persona information "I like marathon races" associated with the user 10 in a data table 244. The determination device 100 holds a deletion history of persona information "I dislike marathon races" associated with the user 10 in a data table 246.

In this case, when the user 10 utters "I'm looking forward participating a marathon race" to the interaction system 230, the interaction system 230 refers to the data tables 244 and 246. Then, the interaction system 230 refers to the persona information and deletion history on the basis of a content (marathon) of the topic talked about and the like, and outputs an answer reflecting past information and current persona information such as "I did not like it before, but I got into it once I tried" on the basis of such information.

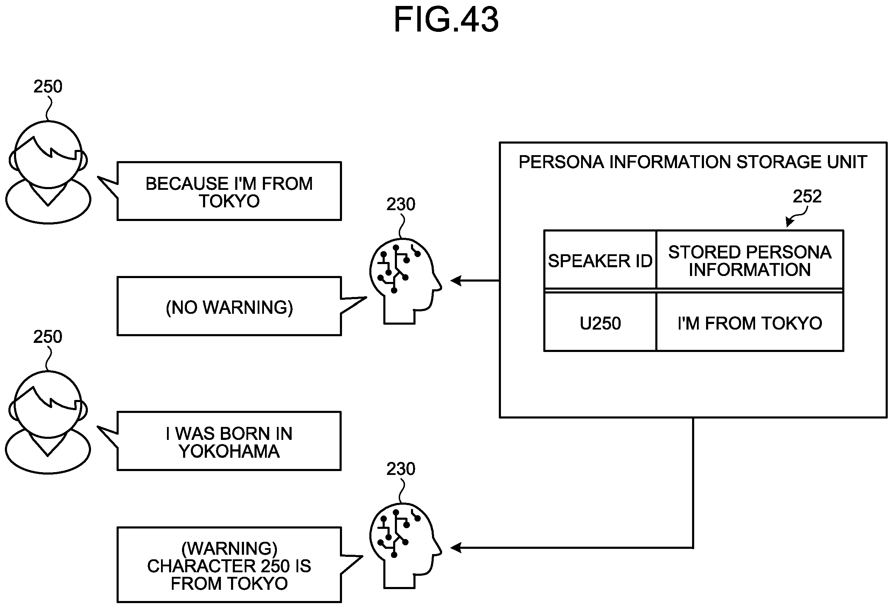

FIG. 43 is a diagram (7) illustrating a specific example of determination processing according to the third embodiment. FIG. 43 illustrates a case where the interaction system 230 operates as a line supervision support application that checks, for example, a drama script. In this case, the determination device 100 holds persona information associated with a character to be checked in a data table 252. Note that, in the example of FIG. 43, it is assumed that the character to be checked is a character performed by a user 250.

In this case, when the user 250 utters "Because I'm from Tokyo" to the interaction system 230 as a practice of the line, the interaction system 230 refers to the data table 252. Then, the interaction system 230 determines whether the content uttered by the user 250 and the information held in the data table 252 are not inconsistent with each other. In a case where there is no inconsistency, the interaction system 230 does not pay attention to the line of the user 250 without especially warning.

When the user 250 utters "I was born in Yokohama" to the interaction system 230 as a practice of another line, the interaction system 230 refers to the data table 252. Then, the interaction system 230 determines whether the content uttered by the user 250 and the information held in the data table 252 are not inconsistent with each other. Herein, when determining that the information "I'm from Tokyo", which is the persona information held in the data table 252, is inconsistent from the line, the interaction system 230 issues a warning to correct the persona information.

Specifically, the interaction system 230 issues a warning that "the character 250 is from Tokyo". As a result, the user 250 can confirm the inconsistency occurring in the setting of the character that the user plays.

Note that, in the example as illustrated in FIG. 43, the interaction system 230 may not only warn about the utterance but also receive text data and the like of a newly created script as an input and warn about the inconsistency.

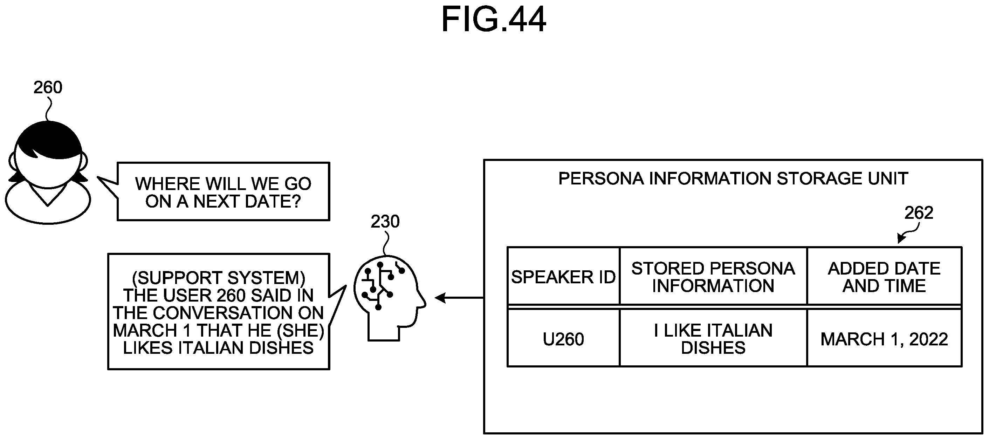

FIG. 44 is a diagram (8) illustrating a specific example of determination processing according to the third embodiment. FIG. 44 illustrates a case in which the interaction system 230 operates as a communication support function on a message function in a communication application or a matching application.

In this case, the determination device 100 holds persona information associated with a target user 260 in a data table 262. In the example of FIG. 44, the interaction system 230 operates as a support system for a user who tries to communicate with the user 260.

For example, in a case where the user 260 utters "Where will we go on a next date?", the interaction system 230 receives such utterance and refers to the persona information of the user 260. Then, the interaction system 230 creates a supporting message on the basis of taste information and the like of the user 260. Then, the interaction system 230 outputs an advice such as "The user 260 said in the conversation on March 1 that he (she) likes Italian dishes" to the supported user.

Upon receiving such advice, the user can invite the user 260 to go eating Italian dishes. Note that, as illustrated in FIG. 44, the interaction system 230 may operate to reduce a labor of confirming message exchange serving as the basis of the persona information by including, in a notification sentence, information of a time stamp when the persona information "I like Italian dishes" is stored.

### (4. Fourth Embodiment)

Next, a fourth embodiment is described. In the fourth embodiment, a determination device 100 performs processing of integrating held persona information.

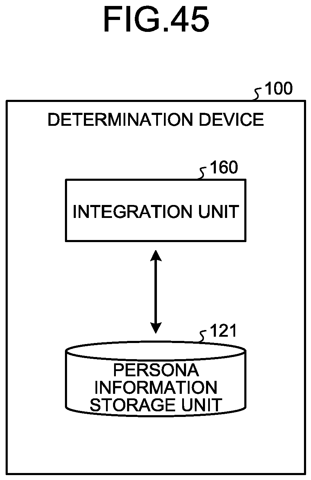

FIG. 45 is a diagram illustrating a configuration example of the determination device 100 according to the fourth embodiment. As illustrated in FIG. 45, the determination device 100 includes an integration unit 160.

In a case where a plurality of pieces of persona information of a user updated by an update unit 134 is held, the integration unit 160 integrates the plurality of pieces of persona information.

For example, the integration unit 160 provides the user with information serving as a basis for integrating the plurality of pieces of persona information via a user interface, and integrates the plurality of pieces of persona information designated by the user in the user interface.

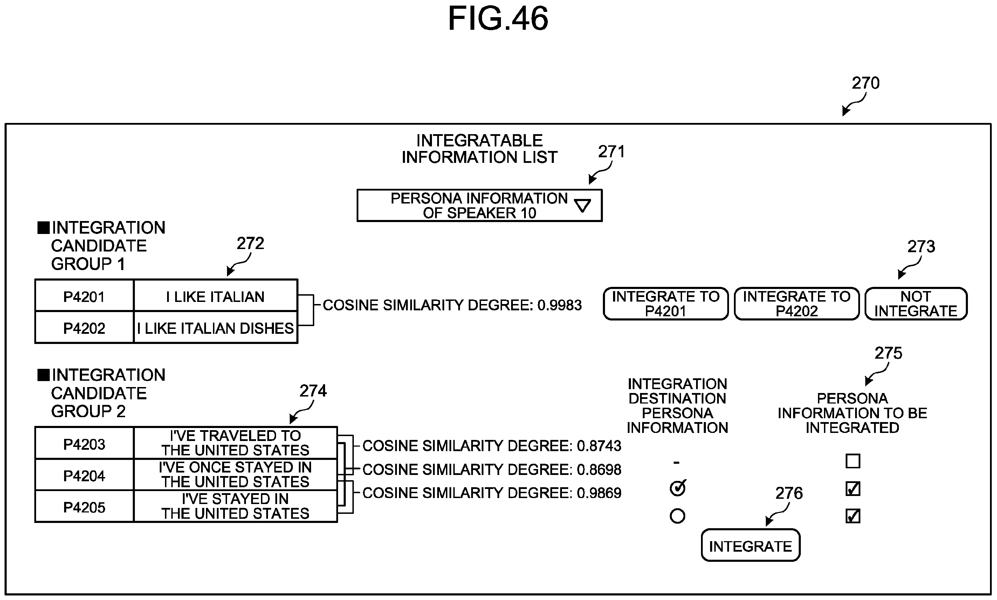

A specific example of the fourth embodiment is described with reference to FIG. 46. FIG. 46 is a diagram illustrating a specific example of determination processing according to the fourth embodiment.

FIG. 46 illustrates a user interface 270 provided by the determination device 100. The user can confirm the persona information stored in association with the user by selecting the user in a selection window 271.

At that time, as illustrated in FIG. 46, the determination device 100 provides a similarity degree on the basis of attributes and contents of the pieces of persona information out of the persona information held in association with the user 10. For example, the determination device 100 indicates a cosine similarity degree indicating a similarity degree between sentences. For example, the determination device 100 provides the cosine similarity degree of integration candidate persona information 272 and the cosine similarity degree of integration candidate persona information 274.

In a case of desiring the integration, the user 10 may integrate persona information by pressing a decision button 273. In a case of integrating a plurality of pieces of persona information, the user 10 can select a plurality of pieces of persona information to be integrated using a check box 275, and selects one piece of integration destination persona information using a radio button. In this manner, in a case of integrating a plurality of pieces of persona information, the user 10 can perform intended persona information integration by selecting the persona information to be integrated and the integration destination persona information to select integration, and pressing a decision button 276.

As a result, the user 10 can integrate similar pieces of persona information and summarize the information briefly. Note that, such integration may be automatically performed by the determination device 100 on the basis of cosine similarity degree and the like.

The determination device 100 may calculate similarity degree scores such as cosine similarity degree by the persona information on a one-to-one basis or on a one-to-many basis. Such calculation may be rule processing, or machine learning may be used.

### (5. Fifth Embodiment)

Next, a fifth embodiment is described. In the fifth embodiment, a determination device 100 receives a parameter that can be optionally changed from a user, and performs processing of generating persona information on the basis of the received parameter.

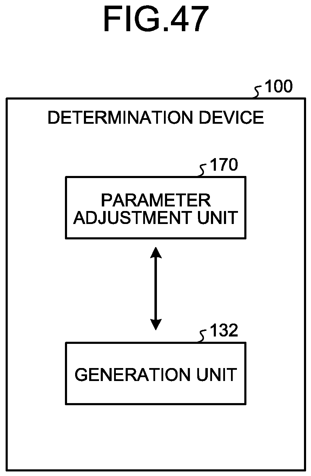

FIG. 47 is a diagram illustrating a configuration example of the determination device 100 according to the fifth embodiment. As illustrated in FIG. 47, the determination device 100 according to the fifth embodiment includes a parameter adjustment unit 170.

The parameter adjustment unit 170 receives reaction information in generation of first persona information and adjustment of a weight of user information acquired together with the reaction information, from the user via the user interface. In this case, the generation unit 132 generates the first persona information on the basis of the weight adjusted by the parameter adjustment unit 170.

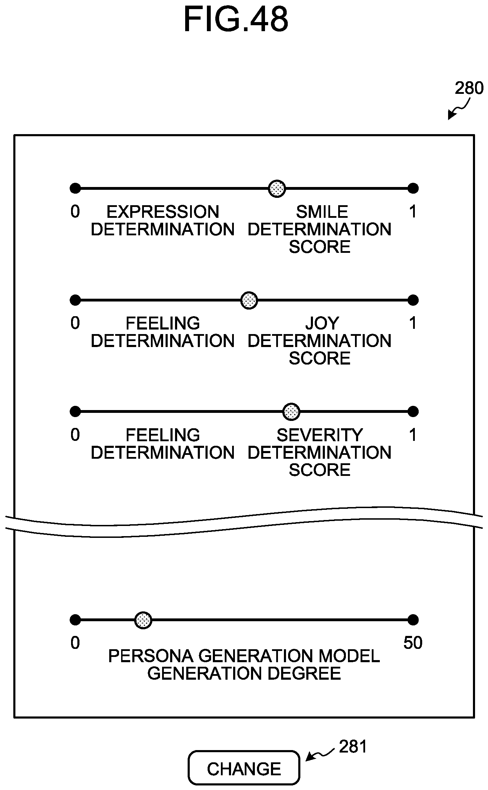

A specific example of the fifth embodiment is described with reference to FIG. 48 and subsequent drawings. FIG. 48 is a diagram (1) illustrating a specific example of determination processing according to the fifth embodiment. FIG. 48 illustrates a user interface 280 provided by the determination device 100.

The user interface 280 includes a slider in each item so that the user can optionally adjust a weight of score used for expression determination and a weight of score used for feeling determination. For example, in a case where the user wants to increase the weight of severity determination score of the feeling determination out of the information used for the persona information, the user moves the slider rightward in the user interface 280. As a result, when generating the persona information, the determination device 100 generates the persona information by weighting the severity determination score of the feeling determination.

Note that, the user interface 280 illustrated in FIG. 48 is an example, and the determination device 100 may provide a user interface of various modes such as an input means other than the slider such as a radio button, and may enable a direct input of the parameter by preparing a text box.

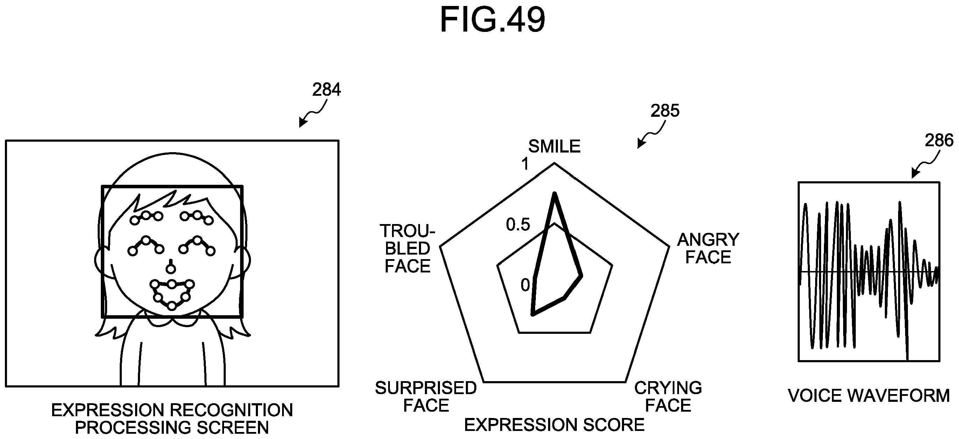

The determination device 100 may further provide information to be referred to by the user together with the user interface 280. This point is described with reference to FIG. 49. FIG. 49 is a diagram (2) illustrating a specific example of determination processing according to the fifth embodiment. FIG. 49 illustrates expression determination information 284, expression score 285, and a voice waveform 286 out of elements received by the determination device 100.

For example, the determination device 100 displays the information illustrated in FIG. 49 on a user interface, and indicates which expression score is generated from a feature amount of which expression in generating the persona information. Alternatively, the determination device 100 may indicate which kind of feeling score is generated from which kind of voice waveform in the generation of the persona information.

The user can set a weight desired by the user by adjusting the parameter with reference to the information presented from the determination device 100.

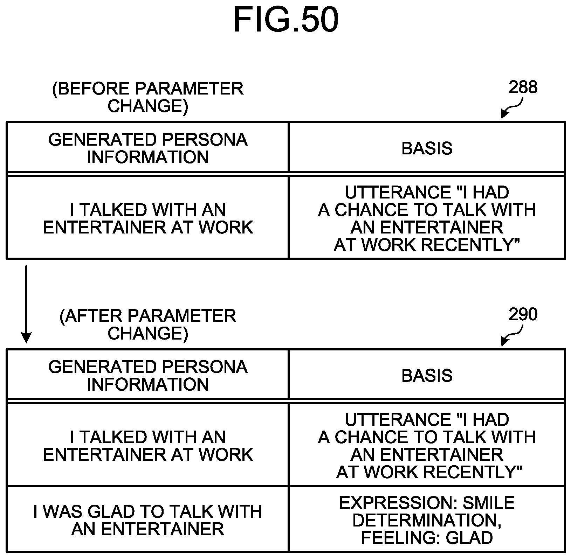

In a case where the user adjusts the parameter, the determination device 100 may provide a preview indicating how the persona information generated before and after the adjustment changes. This point is described with reference to FIG. 50.

FIG. 50 is a diagram (3) illustrating a specific example of determination processing according to the fifth embodiment. FIG. 50 includes a data table 288 including the persona information generated with the weight before the parameter adjustment and a data table 290 including the persona information generated with the weight after the parameter adjustment.

The data table 288 illustrates an example in which, in a case where the user utters "I had a chance to talk with an entertainer at work recently", the determination device 100 generates persona information "I talked with an entertainer at work" indicating only the fact before the parameter adjustment.

Herein, in a case where the user adjusts the parameter so as to increase the weight of smile determination or the feeling determination based on voice, the persona information generated by the determination device 100 changes. For example, the determination device 100 can easily determine smile even with a slight change in expression of the user, persona information based on the smile is further generated.

In the data table 290, in a case where the user utters "I had a chance to talk with an entertainer at work recently", the determination device 100 generates persona information "I was glad to talk with an entertainer" generated on the basis of the expression of the user together with persona information "I talked with an entertainer at work" indicating the fact.

By presenting such a difference in generation processing to the user as a preview, the determination device 100 can notify the user how the user can preferably change the parameter. That is, the user can easily grasp which kind of persona information is generated on the basis of which kind of information when the parameter is changed. As a result, the user can adjust the generation of the persona information, so that the user can bring the interaction system and the like used by him/herself closer to his/her preference.

### (6. Variation)

### (6-1. Device Configuration)

The determination device 100 according to each embodiment merely conceptually illustrates functions, and can take various modes depending on the embodiment. For example, the determination device 100 may include two or more devices different for each of the above-described functions. As an example, the determination device 100 may include a cloud server and an edge terminal (a smart speaker, a smartphone and the like) connected via a network. In this case, when the edge terminal acquires voice, the edge terminal transmits the acquired information to the cloud server. Then, the cloud server performs generation processing and determination processing described in each embodiment, and reflects results thereof in processing executed by the edge terminal.

### (6-2. Reaction Information)

The determination device 100 can acquire not only voice but also various pieces of information as the reaction information of the user. For example, the determination device 100 acquires behavior information on the web as the reaction information of the user. As an example, in a case where the user purchases or browses a product on a shopping site and the like, the determination device 100 determines that the user tends to like such product, and generates such persona information. Alternatively, the determination device 100 acquires a content of the browsed web page and the like, determines that the user tends to like such content, and generates such persona information. Alternatively, in a case where the user frequently browses a web page of a predetermined organization, the determination device 100 may generate persona information indicating that the user belongs to the organization.

Then, in a case where the user performs a behavior different from the held persona information, the determination device 100 may determine consistency thereof and update the persona information. In this manner, since the determination device 100 can update the persona information in real time on the basis of the user's behavior on the network, it is possible to set the persona information appropriately reflecting the user's latest behavior.

### (7. Other Embodiment)

The processing according to each embodiment described above may be performed in various different modes in addition to each embodiment described above.

Out of each processing described in the above-described embodiments, an entire or a part of the processing described as being performed automatically can be performed manually, or an entire or a part of the processing described as being performed manually can be performed automatically by a known method. The procedure, specific names, and information including various data and parameters described in the document and illustrated in the drawings can be optionally changed unless otherwise specified. For example, the various types of information illustrated in each drawing are not limited to the illustrated information.

Each component of each device illustrated in the drawings is functionally conceptual, and is not necessarily physically configured as illustrated in the drawings. That is, a specific form of distribution and integration of each device is not limited to the illustrated form, and an entire or a part thereof can be functionally or physically distributed and integrated in any unit according to various loads, usage conditions and the like. For example, the generation unit 132 and the determination unit 133 may be integrated.

The above-described embodiments and variations can be appropriately combined within a range in which there is no inconsistency in processing contents.

The effects described in the present specification are merely examples and are not limited, and there may be other effects.

### (8. Effect of Determination Device according to Present Disclosure)

As described above, the determination device (the determination device 100 in the embodiment) according to the present disclosure includes the input unit (the input unit 131 in the embodiment), the generation unit (the generation unit 132 in the embodiment), the determination unit (the determination unit 133 in the embodiment), and the update unit (the update unit 134 in the embodiment). The input unit receives the input of the reaction information indicating the reaction of the user. The generation unit generates the first persona information that is the information generated on the basis of the reaction information received by the input unit and indicates the characteristic of the user. The determination unit determines the consistency between the first persona information generated by the generation unit and the second persona information based on the past reaction information of the user. The update unit updates the persona information related to the user on the basis of the consistency determined by the determination unit.

In this manner, the determination device according to the present disclosure determines consistency with past persona information in a case of generating the persona information indicating personal information of the individual user. That is, since the determination device can hold only consistent information as the persona information, it is possible to appropriately determine information indicating a characteristic of the individual user.

In a case where there is the inconsistency between the first persona information and the second persona information, the determination unit transmits a question regarding the inconsistency to the user, and determines the consistency on the basis of an answer of the user.

In this manner, the determination device generates a question for determining consistency, and determines consistency on the basis of the answer, so that highly reliable persona information can be held.

Alternatively, in a case where the determination unit determines that the first persona information and the second persona information are not consistent, the update unit holds either the first persona information or the second persona information as the persona information of the user, and discards the persona information that is not held.

In this manner, since the determination device automatically discards the inconsistent persona information, it is possible to hold only appropriate persona information without a labor for the user to manually select the persona information.

The generation unit gives basis information, which is information serving as a basis for generating the first persona information, to the first persona information. When generating the first persona information, the generation unit classifies the first persona information according to type.

In this manner, by giving the basis information when generating the persona information, the determination device can cause the user to easily confirm the element on the basis of which the persona information is generated.

The generation unit generates the first persona information on the basis of the reaction information and the information held in a predetermined knowledge database (the persona knowledge database 122 in the embodiment).

In this manner, the determination device can automatically generate the persona information inferred by the knowledge by performing the generation processing including the external knowledge data. As a result, the determination device can implement more natural interaction and can implement proactive information processing based on the inferred information.

The determination device further includes the utilization unit (the utilization unit 140 in the embodiment) that provides the persona information regarding the user updated by the update unit to a predetermined external program to utilize.

In this manner, by utilizing the persona information in the external application, the determination device can cause the application to execute various pieces of processing on the premise of the attribute information of the user and the like.

The determination device further includes the integration unit (the integration unit 160 in the embodiment) that integrates, in a case where a plurality of pieces of persona information of the user updated by the update unit is held, the plurality of pieces of persona information. For example, the integration unit provides the user with information serving as a basis for integrating the plurality of pieces of persona information via a user interface, and integrates the plurality of pieces of persona information designated by the user in the user interface.

In this manner, the determination device may organize unnecessary persona information and hold only more appropriate persona information by integrating the persona information.

The determination device further includes the adjustment unit (the parameter adjustment unit 170 in the embodiment) that receives the reaction information in the generation of the first persona information and adjustment of the weight of the user information acquired together with the reaction information, from the user via the user interface. The generation unit generates the first persona information on the basis of the weight adjusted by the adjustment unit.

In this manner, the determination device can implement the generation processing in a mode more preferred by the user by receiving the parameter adjustment for adjusting the generation of the persona information.

The input unit receives the input of the voice uttered by the user or the text corresponding to the voice as the reaction information. The generation unit generates the first persona information on the basis of at least one of the volume of the voice uttered by the user, the feeling of the user estimated from the voice, the conversation speed in the voice, or the surrounding environmental sound.

In this manner, since the determination device generates the persona information by estimating not only the voice-recognized text but also the feeling and the like when the voice is uttered, it is possible to generate the persona information reflecting not only superficial persona information but also the user's feeling and the like.

The input unit receives an input of image information obtained by imaging the user together with the reaction information. The generation unit generates the first persona information using at least one of the expression, motion, and line of sight of the user estimated from the image information, or the object information included in the image information.

In this manner, since the determination device generates the persona information on the basis of the expression and the like of the user at the time of utterance, it is possible to generate the persona information further reflecting the feeling and the like of the user.

The input unit receives an input of biological information detected from the user together with the reaction information. The generation unit generates the first persona information by using at least one piece of information of the perspiration amount, body temperature, heartbeat, pulse, blood pressure, or respiratory rate of the user measured as the biological information.

In this manner, since the determination device generates the persona information on the basis of the biological information and the like of the user at the time of utterance, it is possible to generate the persona information reflecting fluctuation in feeling and the like of the user, which is not perceived by the user.

The input unit receives the text input by the user as the reaction information. Alternatively, the input unit receives the behavior information on the network of the user as the reaction information.

In this manner, the determination device can generate the persona information based on not only the voice but also various behaviors of the user, and determine consistency. As a result, the determination device can update the persona information in real time in accordance with the user behavior.

### (9. Hardware Configuration)

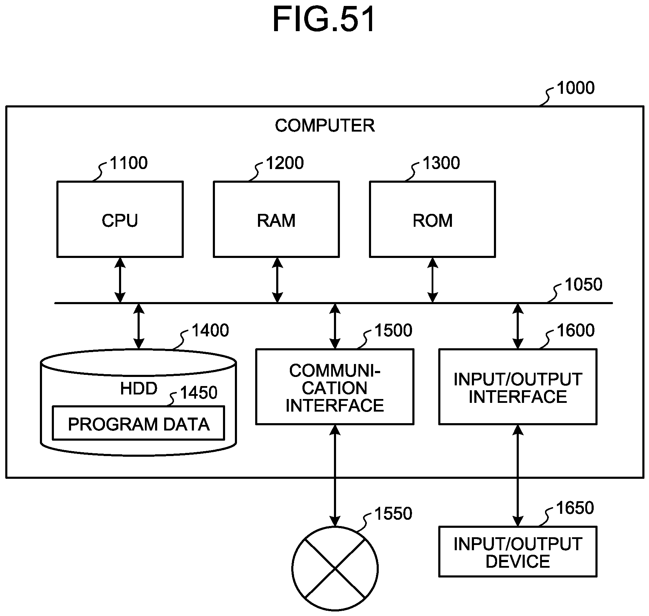

The information device such as the determination device 100 according to each embodiment described above is implemented by a computer 1000 having a configuration as illustrated in FIG. 51, for example. Hereinafter, the determination device 100 is described as an example. FIG. 51 is a hardware configuration diagram illustrating an example of the computer 1000 that implements functions of the determination device 100. The computer 1000 includes a CPU 1100, a RAM 1200, a read only memory (ROM) 1300, a hard disk drive (HDD) 1400, a communication interface 1500, and an input/output interface 1600. Each unit of the computer 1000 is connected by a bus 1050.

The CPU 1100 operates on the basis of a program stored in the ROM 1300 or the HDD 1400, and controls each unit. For example, the CPU 1100 develops the program stored in the ROM 1300 or the HDD 1400 in the RAM 1200, and executes processing corresponding to various programs.

The ROM 1300 stores a boot program such as a basic input output system (BIOS) executed by the CPU 1100 when the computer 1000 is activated, a program depending on hardware of the computer 1000 and the like.

The HDD 1400 is a computer-readable recording medium that non-transiently records a program executed by the CPU 1100, data used by the program and the like. Specifically, the HDD 1400 is a recording medium that records a determination program according to the present disclosure as an example of program data 1450.

The communication interface 1500 is an interface for the computer 1000 to connect to an external network 1550 (for example, the Internet). For example, the CPU 1100 receives data from another device or transmits data generated by the CPU 1100 to another device via the communication interface 1500.

The input/output interface 1600 is an interface for connecting an input/output device 1650 and the computer 1000. For example, the CPU 1100 receives data from an input device such as a keyboard and a mouse via the input/output interface 1600. The CPU 1100 also transmits data to an output device such as a display, an edgy, or a printer via the input/output interface 1600. The input/output interface 1600 may function as a medium interface that reads a program and the like recorded in a predetermined recording medium (medium). The medium is, for example, an optical recording medium such as a digital versatile disc (DVD) or a phase change rewritable disk (PD), a magneto-optical recording medium such as a magneto-optical disk (MO), a tape medium, a magnetic recording medium, a semiconductor memory or the like.

For example, in a case where the computer 1000 functions as the determination device 100 according to the embodiment, the CPU 1100 of the computer 1000 implements the functions of the control unit 130 and the like by executing the determination program loaded on the RAM 1200. The HDD 1400 stores the determination program according to the present disclosure and data in the storage unit 120. Note that, the CPU 1100 reads the program data 1450 from the HDD 1400 to execute, but as another example, these programs may be acquired from another device via the external network 1550.

Note that, the present technology can also have the following configurations.
(1) A determination device comprising:
   an input unit that receives an input of reaction information indicating a reaction of a user;
   a generation unit that generates first persona information that is information generated on a basis of the reaction information received by the input unit, the information indicating a characteristic of the user;
   a determination unit that determines consistency between the first persona information generated by the generation unit and second persona information based on past reaction information of the user; and
   an update unit that updates the persona information regarding the user on a basis of the consistency determined by the determination unit.
(2) The determination device according to (1), wherein
   in a case where there is inconsistency between the first persona information and the second persona information, the determination unit transmits a question regarding the inconsistency to the user, and determines the consistency on a basis of an answer of the user.
(3) The determination device according to (1) or (2), wherein
   in a case where the determination unit determines that the first persona information and the second persona information are not consistent, the update unit holds either the first persona information or the second persona information as the persona information of the user, and discards the persona information that is not held.
(4) The determination device according to any one of (1) to (3), wherein
   the generation unit gives basis information, which is information serving as a basis for generating the first persona information, to the first persona information.
(5) The determination device according to any one of (1) to (4), wherein
   when generating the first persona information, the generation unit classifies the first persona information according to type.
(6) The determination device according to any one of (1) to (5), wherein
   the generation unit generates the first persona information on a basis of the reaction information and information held in a predetermined knowledge database.
(7) The determination device according to any one of (1) to (6), further comprising:
   a utilization unit that provides the persona information regarding the user updated by the update unit to a predetermined external program to utilize.
(8) The determination device according to any one of (1) to (7), further comprising:
   an integration unit that integrates, in a case where a plurality of pieces of persona information of the user updated by the update unit is held, the plurality of pieces of persona information.
(9) The determination device according to (8), wherein
   the integration unit provides the user with information serving as a basis for integrating the plurality of pieces of persona information via a user interface, and integrates the plurality of pieces of persona information designated by the user in the user interface.
(10) The determination device according to any one of (1) to (9), further comprising:
   an adjustment unit that receives the reaction information in generation of the first persona information and adjustment of a weight of information of the user acquired together with the reaction information, from the user via a user interface, wherein
   the generation unit generates the first persona information on a basis of the weight adjusted by the adjustment unit.
(11) The determination device according to any one of (1) to (10), wherein
   the input unit receives an input of voice uttered by the user or a text corresponding to the voice.
(12) The determination device according to (11), wherein
   the generation unit generates the first persona information on a basis of at least one of a volume of the voice uttered by the user, feeling of the user estimated from the voice, a conversation speed in the voice, or a surrounding environmental sound.
(13) The determination device according to any one of (1) to (12), wherein
   the input unit receives an input of image information obtained by imaging the user together with the reaction information, and
   the generation unit generates the first persona information by using at least one of expression, motion, and a line of sight of the user estimated from the image information, or object information included in the image information.
(14) The determination device according to any one of (1) to (13), wherein
   the input unit receives an input of biological information detected from the user together with the reaction information, and
   the generation unit generates the first persona information by using at least one piece of information of a perspiration amount, body temperature, heartbeat, pulse, blood pressure, or respiratory rate of the user measured as the biological information.
(15) The determination device according to any one of (1) to (14), wherein
   the input unit receives a text input by the user as the reaction information.
(16) The determination device according to any one of (1) to (15), wherein
   the input unit receives behavior information on a network of the user as the reaction information.
(17) A determination method causing
   a computer to execute the steps of:
   receiving an input of reaction information indicating a reaction of a user;
   generating first persona information that is information generated on a basis of the received reaction information, the information indicating a characteristic of the user;
   determining consistency between the generated first persona information and second persona information based on past reaction information of the user; and
   updating the persona information regarding the user on a basis of the determined consistency.

### Reference Signs List

- 10: USER

- 100: DETERMINATION DEVICE
- 110: COMMUNICATION UNIT
- 120: STORAGE UNIT
- 121: PERSONA INFORMATION STORAGE UNIT
- 122: PERSONA KNOWLEDGE DATABASE
- 130: CONTROL UNIT
- 131: INPUT UNIT
- 132: GENERATION UNIT
- 133: DETERMINATION UNIT
- 134: UPDATE UNIT
- 135: OUTPUT UNIT
- 140: UTILIZATION UNIT
- 150: APPLICATION
- 160: INTEGRATION UNIT
- 170: PARAMETER ADJUSTMENT UNIT

## Claims

1. A determination device comprising:
an input unit that receives an input of reaction information indicating a reaction of a user;
a generation unit that generates first persona information that is information generated on a basis of the reaction information received by the input unit, the information indicating a characteristic of the user;
a determination unit that determines consistency between the first persona information generated by the generation unit and second persona information based on past reaction information of the user; and
an update unit that updates the persona information regarding the user on a basis of the consistency determined by the determination unit.

2. The determination device according to claim 1, wherein
in a case where there is inconsistency between the first persona information and the second persona information, the determination unit transmits a question regarding the inconsistency to the user, and determines the consistency on a basis of an answer of the user.

3. The determination device according to claim 1, wherein
in a case where the determination unit determines that the first persona information and the second persona information are not consistent, the update unit holds either the first persona information or the second persona information as the persona information of the user, and discards the persona information that is not held.

4. The determination device according to claim 1, wherein
the generation unit gives basis information, which is information serving as a basis for generating the first persona information, to the first persona information.

5. The determination device according to claim 1, wherein
when generating the first persona information, the generation unit classifies the first persona information according to type.

6. The determination device according to claim 1, wherein
the generation unit generates the first persona information on a basis of the reaction information and information held in a predetermined knowledge database.

7. The determination device according to claim 1, further comprising:
a utilization unit that provides the persona information regarding the user updated by the update unit to a predetermined external program to utilize.

8. The determination device according to claim 1, further comprising:
an integration unit that integrates, in a case where a plurality of pieces of persona information of the user updated by the update unit is held, the plurality of pieces of persona information.

9. The determination device according to claim 8, wherein
the integration unit provides the user with information serving as a basis for integrating the plurality of pieces of persona information via a user interface, and integrates the plurality of pieces of persona information designated by the user in the user interface.

10. The determination device according to claim 1, further comprising:
an adjustment unit that receives the reaction information in generation of the first persona information and adjustment of a weight of information of the user acquired together with the reaction information, from the user via a user interface, wherein
the generation unit generates the first persona information on a basis of the weight adjusted by the adjustment unit.

11. The determination device according to claim 1, wherein
the input unit receives an input of voice uttered by the user or a text corresponding to the voice.

12. The determination device according to claim 11, wherein
the generation unit generates the first persona information on a basis of at least one of a volume of the voice uttered by the user, feeling of the user estimated from the voice, a conversation speed in the voice, or a surrounding environmental sound.

13. The determination device according to claim 1, wherein
the input unit receives an input of image information obtained by imaging the user together with the reaction information, and
the generation unit generates the first persona information by using at least one of expression, motion, and a line of sight of the user estimated from the image information, or object information included in the image information.

14. The determination device according to claim 1, wherein
the input unit receives an input of biological information detected from the user together with the reaction information, and
the generation unit generates the first persona information by using at least one piece of information of a perspiration amount, body temperature, heartbeat, pulse, blood pressure, or respiratory rate of the user measured as the biological information.

15. The determination device according to claim 1, wherein
the input unit receives a text input by the user as the reaction information.

16. The determination device according to claim 1, wherein
the input unit receives behavior information on a network of the user as the reaction information.

17. A determination method causing
a computer to execute the steps of:
receiving an input of reaction information indicating a reaction of a user;
generating first persona information that is information generated on a basis of the received reaction information, the information indicating a characteristic of the user;
determining consistency between the generated first persona information and second persona information based on past reaction information of the user; and
updating the persona information regarding the user on a basis of the determined consistency.
